(19) Europäisches Patentamt
European Patent Office
Office européen des brevets

(11) **EP 3 438 243 B1**

(12) **EUROPEAN PATENT SPECIFICATION**

(45) Date of publication and mention
of the grant of the patent:
**17.11.2021 Bulletin 2021/46**

(21) Application number: **17775506.3**

(22) Date of filing: **30.03.2017**

(51) Int Cl.:
*C12N 1/02* *(2006.01)*        *B01D 65/06* *(2006.01)*
*B01D 69/00* *(2006.01)*       *B01D 71/32* *(2006.01)*
*C12C 7/16* *(2006.01)*        *C12H 1/16* *(2006.01)*
*C12M 1/12* *(2006.01)*        *B01D 61/14* *(2006.01)*
*B01D 65/02* *(2006.01)*

(86) International application number:
**PCT/JP2017/013492**

(87) International publication number:
**WO 2017/170973 (05.10.2017 Gazette 2017/40)**

(54) **METHOD FOR FILTERING MICROBIAL CULTURE SOLUTION USING MEMBRANE MODULE**

VERFAHREN ZUM FILTERN EINER BAKTERIENKULTURLÖSUNG MIT EINEM MEMBRANMODUL

PROCÉDÉ DE FILTRATION D'UNE SOLUTION DE CULTURE MICROBIENNE À L'AIDE D'UN MODULE À MEMBRANE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **30.03.2016 JP 2016068781**

(43) Date of publication of application:
**06.02.2019 Bulletin 2019/06**

(73) Proprietor: **Toray Industries, Inc.**
**Tokyo 103-8666 (JP)**

(72) Inventors:
• **SHIMURA, Fumi**
  **Otsu-shi**
  **Shiga 520-8558 (JP)**
• **SHIMURA, Shun**
  **Otsu-shi**
  **Shiga 520-8558 (JP)**
• **KOBAYASHI, Atsushi**
  **Otsu-shi**
  **Shiga 520-8558 (JP)**
• **NISHIO, Aya**
  **Otsu-shi**
  **Shiga 520-8558 (JP)**
• **KANAMORI, Satoko**
  **Otsu-shi**
  **Shiga 520-8558 (JP)**

(74) Representative: **Kador & Partner PartG mbB**
**Corneliusstraße 15**
**80469 München (DE)**

(56) References cited:
**WO-A1-02/092202        GB-A- 2 124 199**
**JP-A- H0 263 530        JP-A- 2001 504 705**
**JP-A- 2005 537 120      JP-A- 2013 031 839**
**US-A1- 2008 264 454     US-A1- 2013 330 792**

Remarks:
The file contains technical information submitted after the application was filed and not included in this specification

## Description

TECHNICAL FIELD

The present invention relates to a filtration method for filtrating a microbial culture solution using a membrane module in the field of fermentation industry, and the field of food industry.

BACKGROUND ART

For a microbial dispersion solution, a method of separating a microorganism and a solution using a separation membrane is known. As the microbial dispersion solution, activated sludge in wastewater treatment and a microbial culture solution in a fermentation method are mentioned.

In the wastewater treatment, a membrane bioreactor in which activated sludge and treated water are separated from each other using a separation membrane is known. By using the separation membrane, it is possible to maintain a high removal ratio of organic substances and nitrogen components while keeping microorganisms at a high concentration in a treatment tank, and a treated liquid can be obtained at a high purity.

The fermentation method which is a substance production method is used for the production of various products such as beer, wine, vinegar, soy sauce, amino acids, and organic acids. Centrifugal separation or diatomaceous earth filtration is carried out as a method of separating a microbial culture solution in the fermentation method. However, filtration by a separation membrane is characterized by a high separation performance and an excellent filtrate quality.

As described above, a separation technique for the microbial dispersion solution is widely applied. However, in a case where a membrane separation is carried out for a bio-derived liquid to be treated, there is a problem that bacterial cells, sugars, proteins, lipids, and the like contained in the liquid to be treated adhere to a filtration surface and a permeation flux of a membrane decreases at an early stage.

Meanwhile, investigations have been made in which a filtration surface of a membrane is washed in a periodic or non-periodic manner so that adhered matters on the filtration surface are removed and a filtration ability is maintained. For example, Patent Document 1 discloses a method in which a filtration membrane used for membrane bioreactor of wastewater that contains an oil component such as a hydrocarbon compound or an aromatic compound or a hardly decomposable coloring component is washed with chloric acid or a salt thereof and a surfactant. In addition, Patent Document 2 discloses a method in which a separation membrane after beer filtration is washed with a solution containing a periodate compound and peroxydisulfate.

Patent Document 3 discloses a method for producing a chemical by continuous fermentation where the discharged water treatment costs are reduced and the recovery rate of the chemical is enhanced. The method includes a fermentation step of converting a fermentation feedstock to a fermentation liquid containing a chemical, a membrane separation step of recovering a filtrate containing the chemical by a separation membrane from the fermentation liquid; a concentrating step of obtaining a permeate and a concentrate containing the chemical by a reverse osmosis membrane from the filtrate; and a permeate utilization step of using the permeate as at least one of a fermentation feedstock, a pH adjusting solution, a water content adjusting solution for the fermentation liquid, a cleaning solution for the separation membrane and a cleaning solution for the reverse osmosis membrane. The permeate utilization step preferably includes adding any one of an alkali, an acid, and an oxidizing agent to the permeate; and using the permeate after the addition, as a cleaning solution for the separation membrane of the membrane separation step.

Patent Document 4 discloses a method of treating a filter membrane or water-absorbing zone, characterized by contacting the membrane or zone with at least one member selected from the group consisting of an organic solvent, sterilized water containing an organic solvent, sterilized water, enzyme solution containing an adenosine phosphatase, acid, alkali, surfactant, and bleaching agent to remove the adenosine phosphate adherent to the filter membrane or water-absorbing zone.

BACKGROUND ART DOCUMENTS

PATENT DOCUMENTS

[Patent Document 1] JP-A-2013-31839
[Patent Document 2] JP-T-2010-535097
[Patent Document 3] US 2013/330792 A1
[Patent Document 4] WO 02/092202 A1

## SUMMARY OF THE INVENTION

### PROBLEMS THAT THE INVENTION IS TO SOLVE

Among microbial dispersion solutions, in particular, a microbial culture solution has a characteristic feature that concentrations of sugars and proteins are high. When the microbial culture solution is filtrated using a separation membrane, fouling of the separation membrane occurs due to the above-mentioned substances. The fouled separation membrane is caused to restore a water permeability by performing chemical solution washing and can be repeatedly used. However, in a case where the separation membrane is easily fouled, a frequency of the chemical solution washing increases, resulting in an increase of washing costs. In addition, in a case where filtration of the microbial culture solution and chemical solution washing are repeated, fouling substances that cannot be washed accumulate gradually in the separation membrane, which makes it difficult to restore the water permeability.

In the washing method described in Patent Document 1, contaminants on the filtration membrane can be removed by using chloric acid or a salt thereof and a surfactant. However, a liquid to be treated is wastewater containing an oil component and a coloring component, which is different from a microbial culture solution having high concentrations of sugars and proteins.

In addition, the washing method described in Patent Document 2 can be applied to a membrane used for the production of a liquid with high concentrations of sugars and proteins. However, a washing agent to be used is expensive, and as chemical solution washing is repeated, washing costs increase.

The present invention has been made in view of the above. That is, an object of the present invention is to provide a method for filtrating a microbial culture solution using a membrane module, in which a separation membrane used for filtration of a microbial culture solution having high concentrations of sugars and proteins is washed with an inexpensive washing agent to allow filtration to be carried out stably and repeatedly for a long period of time.

### MEANS FOR SOLVING THE PROBLEMS

In order to solve the above problems, the present invention provides a method for filtrating a microbial culture solution using a membrane module as defined in claim 1 or claim 7.Preferred embodiments are defined in the dependent claims.

### ADVANTAGES OF THE INVENTION

The method for filtrating a microbial culture solution using a membrane module of the present invention is a filtration method in which the four steps of (a) performing cross-flow filtration of a microbial culture solution, (b) performing water rinsing, (c) performing chemical solution washing, and (d) performing water rinsing are repeated in this order. Here, in the step c of the chemical solution washing, the washing with a combination of hypochlorite and a nonionic surfactant provides a high washing effect and enables stable filtration over a long period of time.

### BRIEF DESCRIPTION OF THE DRAWINGS

FIG. 1 is a schematic diagram of a filtration apparatus according to an embodiment of the present invention.

FIG. 2 is a flowchart showing an example of the method for filtrating a microbial culture solution of the present invention.

FIG. 3 is a schematic diagram showing a relationship between an F value and a CT value with respect to a filtrate that has permeated through a membrane module according to the embodiment of the present invention, in which the F value is a ratio of a protein concentration [mg/L] in the filtrate with respect to a total organic carbon amount [mg/L] in the filtrate.

FIG. 4 is a schematic diagram showing a change in membrane break strength of a separation membrane, which is a separation membrane according to the embodiment of the present invention, with respect to an immersion time in a Fenton's reagent.

FIG. 5 is a schematic diagram showing a change in membrane strength initial value ratio of a separation membrane, which is a separation membrane according to the embodiment of the present invention, with respect to an immersion time in the Fenton's reagent.

FIG. 6 is a schematic diagram showing a relationship between an attenuation rate $\alpha$ of a separation membrane, which is a separation membrane according to the embodiment of the present invention, and a CT value.

MODE FOR CARRYING OUT THE INVENTION

Hereinafter, a filtration method using a membrane module according to the present invention will be described in detail with reference to the drawings. In the present invention, "upward" and "downward" are based on a state shown in the drawings and are for convenience's sake. A side where a raw liquid flows in is referred to as a "downward" direction, and a side where a filtrate flows out is referred to as an "upward" direction. Usually, in a posture at the time of using a membrane module, upward and downward directions coincide with upward and downward directions in the drawings.

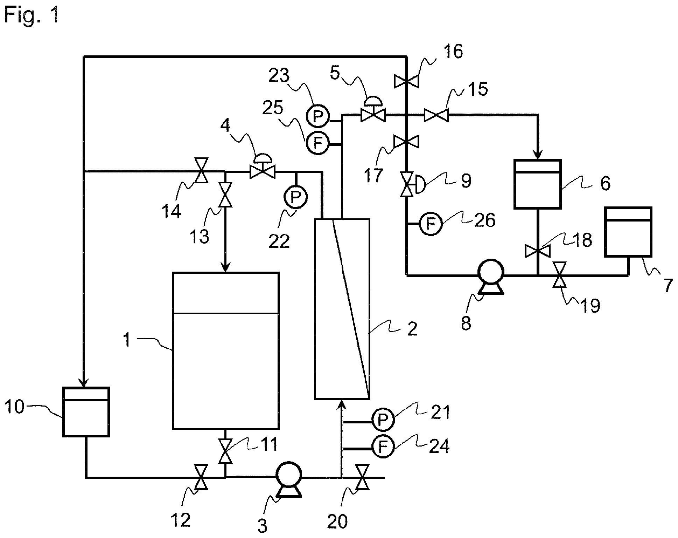

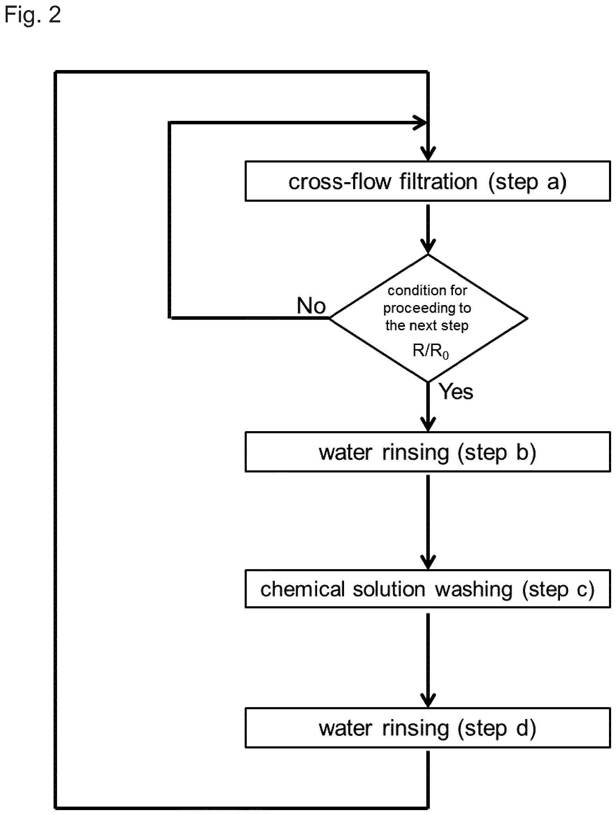

A configuration of a filtration apparatus for a microbial culture solution according to the embodiment of the present invention will be described with reference to the drawings. FIG. 1 is a schematic diagram of a filtration apparatus according to an embodiment of the present invention. FIG. 2 is a flowchart showing an example of the method for filtrating a microbial culture solution of the present invention.

First, an outline of all steps will be described using the flowchart of FIG. 2.

The method for filtrating a microbial culture solution using a membrane module according to the present invention includes the following steps a to d, and the steps a to d are repeated in this order to allow filtration to be carried out stably and repeatedly for a long period of time.

(a) a step of performing cross-flow filtration of a microbial culture solution using a membrane module
(b) a step of performing water rinsing of the membrane module after the step a
(c) a step of bringing the membrane module into contact with a chemical solution after the step b
(d) a step of performing water rinsing of the membrane module after the step c

In the step a, which is the first step, the cross-flow filtration of a microbial culture solution is performed using the membrane module. In this step a, a determination is made on whether or not a filtration resistance value R satisfies a condition for proceeding to the next step. In a case where the condition for proceeding is satisfied, the step proceeds to the step b, and in a case where the condition for proceeding is not satisfied, the cross-flow filtration in the step a is continued.

In the step b, washing of the membrane module is carried out by performing water rinsing. When the step b is completed, the step proceeds to the step c, where chemical solution washing of the membrane module is performed. When the step c is completed, water rinsing in the step d is performed. When the step d is completed, an operation returns back to the step a. The steps a to d are repeated in this order, to carry out filtration of the microbial culture solution. In the present invention, it is sufficient to include the above-mentioned steps a to d, and, as necessary, it is possible to add steps other than the steps a to d.

<Raw liquid>

The microbial culture solution of the present invention is not particularly limited in terms of types of a microorganism and a fermentation product, and examples thereof include beer, wine, vinegar, soy sauce, amino acid fermented liquids, organic acid fermented liquids, and fermented liquids of biopharmaceuticals.

A characteristic feature of the microbial culture solution of the present invention is a microbial dispersion solution in which a filtrate that has permeated through the membrane module by the cross-flow filtration in the step a has a total sugar concentration of 1,000 mg/L to 100,000 mg/L and a protein concentration of 50 mg/L to 1,000 mg/L.

Meanwhile, for the total sugar concentration and the protein concentration in a raw liquid of the microbial culture solution before permeating through the membrane module, due to a pore diameter and a structure of a separation membrane contained in the membrane module, there may be a case of having the same concentrations as the filtrate that has permeated, and a case of having different concentrations as the filtrate that has permeated. When a part of the components is blocked by the separation membrane, concentrations of the components in the filtrate are decreased with respect to concentrations of the components in the raw liquid.

(Step a)

<Cross-flow Filtration>

The microbial culture solution contains various organic substances such as a microorganism, a protein, a saccharide, and lipid, and fouling easily progresses in a dead-end filtration that filtrates all liquids supplied to a membrane module. Therefore, cross-flow filtration in which filtration is carried out by flowing the microbial culture solution as a raw liquid parallel to a membrane surface while washing the membrane surface is suitable (step (a-1) of performing cross-flow filtration of the microbial culture solution using the membrane module).

As shown in FIG. 1, when performing the cross-flow filtration, a microbial culture solution (raw liquid) is supplied from a raw liquid tank 1 to a membrane module 2 by a raw liquid pump 3, and a part of the raw liquid flows back to the raw liquid tank 1. In addition, a filtrate filtrated by a separation membrane of the membrane module 2 is fed to a filtrate tank 6. In order to improve a filtration operability of the microbial culture solution, pretreatment may be carried out before membrane filtration. The pretreatment includes centrifugal separation and adsorption removal.

As described above, in the cross-flow filtration, the raw liquid is circulated between the raw liquid tank 1 and the membrane module 2 in order to allow the raw liquid to flow parallel to the membrane surface. A membrane surface linear velocity at this time may be appropriately set according to properties of the raw liquid, and is 0.1 m/s to 3.0 m/s, and preferably 0.3 m/s to 2.0 m/s. In a case where the membrane surface linear velocity is less than 0.1 m/s, a sufficient washing effect may not be obtained. In addition, in a case where the membrane surface linear velocity exceeds 3.0 m/s, power costs are increased, which is not preferable. A flow rate of the raw liquid can be controlled by the raw liquid pump 3 and a circulation control valve 4.

A filtration flux at the time of performing the cross-flow filtration may be appropriately set according to properties of the raw liquid, and it is 0.1 $m^3/m^2/d$ to 2.0 $m^3/m^2/d$, and preferably 0.3 $m^3/m^2/d$ to 1.5 $m^3/m^2/d$. In a case where the filtration flux is less than 0.1 $m^3/m^2/d$, the number of membrane modules needed is increased, which results in an increase in costs. Meanwhile, in a case where the filtration flux exceeds 2.0 $m^3/m^2/d$, fouling of the separation membrane may progress abruptly, which is not preferable. A flow rate of the filtrate can be controlled by the raw liquid pump 3, the circulation control valve 4, and a filtration control valve 5.

In filtration using the separation membrane, a fouling state of the separation membrane can be determined from an inter-membrane differential pressure obtained by subtracting a pressure on a filtrate side of the separation membrane from a pressure on a raw liquid side thereof. In a case of dead-end filtration, it is possible to calculate the inter-membrane differential pressure from a pressure gauge 21 on a raw liquid inlet side of the membrane module 2 and a pressure gauge 23 on a filtrate outlet side of the membrane module 2. In a case of the same filtration flux, as fouling of the separation membrane progresses, the inter-membrane differential pressure rises. However, in a case of the cross-flow filtration, a large pressure loss occurs when the raw liquid permeates through a raw liquid side flow path of the membrane module 2 and flows back to the raw liquid tank 1, and the above-described calculation method also includes a pressure loss on the raw liquid side flow path. Thus, it is difficult to properly calculate the inter-membrane differential pressure.

Accordingly, in a case where a membrane module filtrate side pressure (a value of the pressure gauge 23) in a case of stopping filtration by causing the filtration control valve 5 to be closed while circulating the raw liquid between the raw liquid tank 1 and the membrane module 2 is denoted by PI, and a membrane module filtrate side pressure (a value of the pressure gauge 23) in a case of carrying out filtration while circulating the raw liquid is denoted by P2, $\Delta$P [Pa], which is a value obtained by subtracting P2 from PI, can be used as a criterion for determining a fouling state in a similar manner to the inter-membrane differential pressure.

In the cross-flow filtration in the step a, as fouling of the separation membrane progresses, the filtration resistance value R rises. When the filtration resistance value R becomes equal to or greater than a certain value, the step proceeds to water rinsing in the step b, and subsequently the step proceeds to chemical solution washing in the step c. However, when the filtration resistance value during performing the cross-flow filtration is set to R [m$^{-1}$] and the filtration resistance value at the start of performing the cross-flow filtration is set to Ro [m$^{-1}$], it is preferable that the step proceeds to the step b when a condition of Expression (2) is satisfied.

The filtration resistance value R [m$^{-1}$] can be calculated from the above-mentioned $\Delta$P [Pa], a filtration flux J [m$^3/m^2/s$], a raw liquid viscosity $\mu$ [Pa · s] by using Expression (3). The filtration resistance value Ro [m$^{-1}$] at the start of performing the filtration is a filtration resistance value at the time when a flow rate and a pressure are stabilized after the start of the filtration, and a filtration resistance value at 60 seconds after operating a valve and a pump to start the filtration may be set as the filtration resistance value Ro [m-1] at the start of performing the filtration.

In a case where fouling of the separation membrane excessively progresses, a water permeability of the separation membrane may not be sufficiently restored even performing the chemical solution washing. Thus, it is preferable that the step proceeds to the step b when R/Ro is equal to or less than 16, and it is more preferable that the step proceeds to the step b in when R/Ro is equal to or less than 12. On the other hand, in a case where the step proceeds to the step b under a condition that $R/R_0$ is less than 5, a frequency of chemical solution washing is increased and costs are increased, which is not preferable.

$$5 \leq R/R_0 \leq 16 \quad \text{Expression (2)}$$

$$R = \Delta P/(J \times \mu) \quad \text{Expression (3)}$$

<Backwashing>

In the cross-flow filtration in the step a, filtration may be stopped in a periodic manner to perform backwashing (step (a-2) of performing backwashing of the membrane module). Once a water permeability is restored by backwashing, a filtration time can be prolonged, and a frequency of chemical solution washing can be decreased so that operating costs are decreased. The filtration resistance value Ro [$m^{-1}$] at the start of performing filtration is a filtration resistance value at the time of starting the filtration for the first time in the step a. In a case where the step a-1 (filtration) and the step a-2 (backwashing) are repeated a plurality of times in the step a, a filtration resistance value at the start of performing filtration in the step a-1 at a first round is set to Ro [$m^{-1}$].

The backwashing may be performed with a filtrate or another liquid such as water may be used. However, in a case where it is not desired to mix another liquid such as water into the raw liquid, it is possible to perform backwashing with the filtrate, or to carry out switching by valve operation and to perform backwashing so that a backwashing liquid is not mixed into the raw liquid.

A backwashing flux at the time of performing the backwashing may be appropriately set according to properties of the raw liquid and a fouling state of the separation membrane, and it is preferably 1.0 $m^3/m^2/d$ to 10.0 $m^3/m^2/d$, and more preferably 1.5 $m^3/m^2/d$ to 5.0 $m^3/m^2/d$. In a case where the backwashing flux is less than 1.0 $m^3/m^2/d$, washing effect is low, which is not preferable. In addition, in a case where the backwashing flux exceeds 10.0 $m^3/m^2/d$, power costs are increased and a large amount of liquids are required for use in backwashing, which is not preferable.

<Recovery of Concentrate >

As the cross-flow filtration is continued in the step a, a liquid amount in the raw liquid tank 1 eventually becomes small, and circulation of the raw liquid between the raw liquid tank 1 and the membrane module 2 becomes difficult. Here, in order to recover the raw liquid remaining in the raw liquid tank, piping, and the membrane module 2, dead-end filtration or diafiltration ma be carried out.

In a case of the dead-end filtration, for example, there may be a method in which water is charged into the raw liquid tank 1, the raw liquid is supplied to the membrane module 2 using the raw liquid pump 3 to perform the dead-end filtration, and the filtration is stopped before the water reaches the membrane module 2. In addition, the dead-end filtration may be carried out by supplying gas to the raw liquid tank 1 and the piping.

In the diafiltration, water is charged into the raw liquid tank 1 and cross-flow filtration is performed, and when a liquid amount in the raw liquid tank 1 is decreased, water is charged again and the cross-flow filtration is repeated so that a recovery ratio of the raw liquid can be improved.

(Step b)

<Water Rinsing>

In the step b, water rinsing of the membrane module 2 with water is performed, which is aimed at reducing contaminant components such as organic substances before the chemical solution washing in the step c. In the chemical solution washing, washing with hypochlorite is carried out. If a large amount of organic substances remain, due to consumption of effective chlorine, the separation membrane may not be sufficiently washed. Thus, before performing the chemical solution washing, water rinsing of the membrane module is performed.

As a method for water rinsing, for example, water is charged into a chemical solution tank 10 and cross-flow filtration of the membrane module 2 is performed, so that the membrane module 2 can be washed. At this time, water is fed from a backwashing liquid tank 7 and backwashing with water is carried out in combination, so that a washing effect can be further increased. For membrane surface linear velocity, filtration flux, and backwashing flux, the water rinsing may be performed under the same conditions as in the step a.

(Step c)

<Chemical Solution Washing >

In the step c, chemical solution washing of the membrane module 2 is performed to restore a water permeability of the separation membrane. The chemical solutions for washing the separation membrane include an acid, an alkali, an oxidizing agent, a surfactant, a chelating agent. Among these, it has been found that washing with a combination of hypochlorite as an oxidizing agent and a nonionic surfactant is effective to wash the separation membrane after filtration of a microbial culture solution with high sugar concentration and protein concentration.

Examples of the hypochlorite include sodium hypochlorite, calcium hypochlorite, and potassium hypochlorite.

For the hypochlorite, one type can be used alone, or two or more types can be used in combination.

In addition, examples of the nonionic surfactant include polyoxyethylene alkyl ether, polyoxyethylene alkyl phenyl ether, polysorbate (polyoxyethylene sorbitan fatty acid ester), polyoxyethylene polystyryl phenyl ether, polyoxyethylene-polyoxypropylene glycol, polyoxyethylene-polyoxypropylene alkyl ether, polyhydric alcohol fatty acid partial ester, polyoxyethylene polyhydric alcohol fatty acid partial ester, polyoxyethylene fatty acid ester, polyglycerin fatty acid ester, and polyoxyethylenated castor oil. For the nonionic surfactant, one type can be used alone, or two or more types can be used in combination.

For a fouling substance such as a protein, a saccharide, and lipid, it is considered that a hydrophobic part in a molecule is attached to the separation membrane. Therefore, an interaction between a hydrophobic part (lipophilic part) of the surfactant and a hydrophobic part of the fouling substance, and a solubility of the surfactant in water affect washing properties. An HLB representing a hydrophilic-lipophilic balance of the nonionic surfactant is 13 to 17. When the HLB is in this range, the surfactant exhibits an excellent balance between hydrophilicity and lipophilicity, and a washing effect is enhanced.

The hypochlorite and the nonionic surfactant are dissolved in water to use as a chemical solution. The hypochlorite and the nonionic surfactant may be used alone, respectively, in a sequential manner or may be used in admixture. Specifically, a method of performing a step (c-1) of bringing into contact with a chemical solution containing the hypochlorite and the nonionic surfactant, or a method of sequentially performing a step (c-2) of bringing into contact with a chemical solution containing the hypochlorite and a step (c-3) of bringing into contact with a chemical solution containing the nonionic surfactant is mentioned. In the step of performing chemical solution washing of the present invention, it is sufficient to include a step in which the hypochlorite and the nonionic surfactant are used alone or in admixture, and a step of washing with another chemical solution may be further included. By including a step of washing with another chemical solution, it is possible to achieve a higher washing restoration ratio.

Because the chemical solution contains both the hypochlorite and the nonionic surfactant as in the step (c-1), a synergistic effect is obtained. In addition, in the step (c-1), the chemical solution contains the hypochlorite and the nonionic surfactant, and may further contain an alkali, an oxidizing agent, an anionic surfactant, a cationic surfactant, an amphoteric surfactant, and a chelating agent.

For a concentration of the hypochlorite used for the chemical solution washing, the hypochlorite has an effective chlorine concentration of 0.05% by mass to 1% by mass, and preferably 0.1% by mass to 0.5% by mass. In a case where the effective chlorine concentration is less than 0.05% by mass, a washing effect may be insufficient. On the other hand, even though the effective chlorine concentration exceeds 1% by mass, a significantly improved washing effect is not recognized, which is disadvantageous from the viewpoint of costs.

Furthermore, it is preferable to change an addition condition of the hypochlorite used for the chemical solution washing in accordance with two parameters of a concentration of a filtrate that has permeated through the membrane module and an oxidation resistance of the separation membrane used. In the present invention, it has been found that a stable operation for a long period of time can be realized by changing the addition condition of the hypochlorite in accordance with the above two parameters.

Specifically, the addition condition is represented by a CT value [(mg/L) · h] that is a product of a concentration C [mg/L] of the hypochlorite in the chemical solution which is in contact with the separation membrane and an inside of the membrane module, and a contact time T[h] between the hypochlorite and the separation membrane.

By selecting the CT value within a range where the separation membrane has an oxidation resistance, it is possible to remove attached fouling substances, and to continue filtration operation for a long period of time without oxidative deterioration of the separation membrane.

On the other hand, in a case where the CT value is high and is exceeding the oxidation resistance of the separation membrane, oxidative deterioration of the separation membrane is accelerated, and a membrane life time is shortened. In addition, in a case where the CT value is low and is insufficient to remove fouling substances attached to the separation membrane, washing effect is insufficient. Here, for the amount of the fouling substances attached to the separation membrane, a concentration of the raw liquid supplied to the membrane module, or a concentration of the filtrate that has permeated through the separation membrane is an index.

Hence, in order to maintain a life time of the separation membrane and to obtain a sufficient washing effect, it is effective to select an appropriate CT value from the two parameters of a concentration of the filtrate and an oxidation resistance of the separation membrane. Methods of selecting the respective parameters will be described in detail below.

The first parameter is a concentration of the filtrate that permeated through the membrane module. As a result of intensive studies, the present inventors have found that by changing the addition condition of the hypochlorite in accordance with a protein concentration in the filtrate, it is possible to obtain a sufficient washing effect regardless of a type of the raw liquid.

That is, when a ratio $Cp/TOC$ of a protein concentration $Cp$ [mg/L] in a filtrate that has permeated through the membrane module by cross-flow filtration in the step a with respect to a total organic carbon amount TOC [mg/L] in the filtrate is represented by F, the CT value is selected within a range that satisfies Expression (4). Furthermore, it is preferable to select the CT value within a range that satisfies Expression (5).

$$9.2 \times 10^5 F - 1400 \leq CT \qquad \text{Expression (4)}$$

$$3.8 \times 10^6 F - 5700 \leq CT \qquad \text{Expression (5)}$$

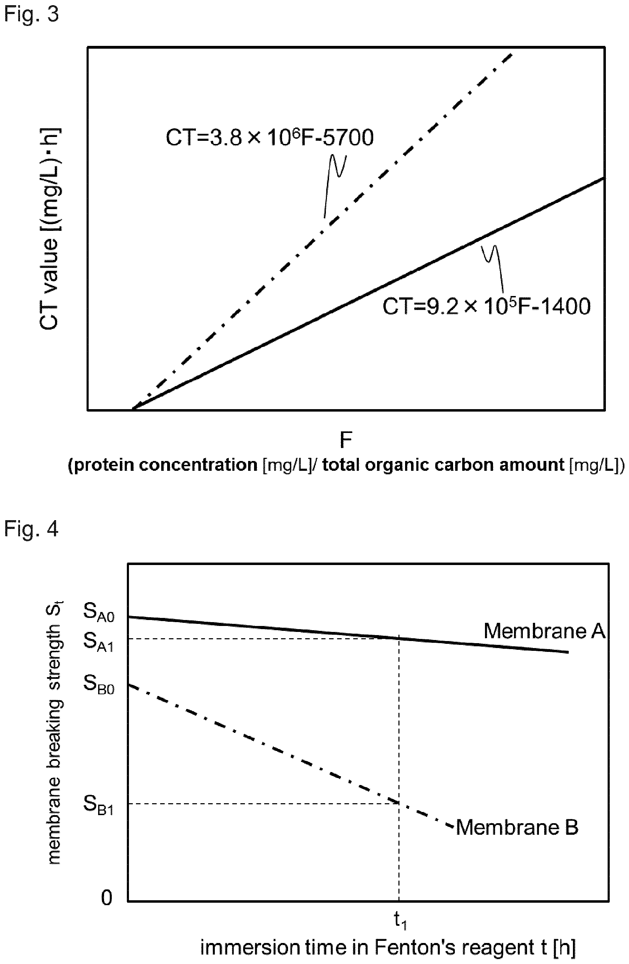

Fig. 3 shows a relationship between the F value and the CT value as represented by Expression (4). In filtration of the microbial culture solution, the higher the F value in the filtrate, the larger an amount of fouling substances to block the separation membrane. Hence, in a case of washing the separation membrane fouled due to filtration of the microbial culture solution, by selecting a CT value in accordance with an F value in the filtrate, too less or too much chemical solution washing is prevented, and, as a result, a high washing effect can be obtained at low costs.

In particular, in the present invention, in a method for filtering a microbial culture solution using a membrane module, in which a filtrate that has permeated through a membrane module has a total sugar concentration of 1,000 mg/L to 100,000 mg/L and a protein concentration of 50 mg/L to 1,000 mg/L, it has been found that by performing chemical solution washing at a CT value that satisfies Expression (4), a sufficient washing effect of the separation membrane can be obtained, and filtration can be repeatedly carried out.

Furthermore, more preferably, by performing chemical solution washing at a CT value that satisfies Expression (5), a higher washing effect can be obtained.

Here, "sufficient washing effect" means that a water permeability restoration rate, which is a rate of a water permeability after the chemical solution washing with respect to a water permeability of an unused membrane before the filtration, is equal to or greater than 80%.

The F value may be represented by a protein concentration in the raw liquid with respect to a total organic carbon amount in the raw liquid.

The second parameter is an oxidation resistance of the separation membrane. The oxidation resistance of the separation membrane varies depending on material and structure thereof. A test method for evaluating the oxidation resistance of the separation membrane includes an accelerated oxidation test in which the separation membrane is immersed in a Fenton's reagent and an amount of change in membrane strength initial value ratio with respect to an immersion time is measured.

The Fenton's reagent is a solution containing hydrogen peroxide and an iron catalyst, and a produced hydroxyl radical exerts a strong oxidizing power. The Fenton's reagent is industrially used for oxidative treatment of contaminated water. In the present invention, the Fenton's reagent is used for the accelerated oxidation test of the separation membrane.

A specific test method is shown below. First, the Fenton's reagent is prepared by adding iron (II) sulfate heptahydrate to hydrogen peroxide. At this time, an $H_2O_2$ concentration is adjusted to 5,000 ppm and a $Fe^{2+}$ concentration is adjusted to 300 ppm. The separation membrane is immersed in the Fenton's reagent which has been adjusted to the above-mentioned concentrations for a certain time period t [h]. The separation membrane is hydrophilized beforehand by a method such as performing immersion in ethanol.

After being immersed in the Fenton's reagent for t [h], the separation membrane is washed with distilled water and vacuum dried at room temperature. A membrane strength is measured for the dried separation membrane (denoted by sample Mt) and a separation membrane (denoted by sample $M_0$) which has been vacuum-dried at room temperature without being immersed in the Fenton's reagent. A method of measuring the membrane strength is not limited, and, for example, a tensile test machine (TENSILON (registered trademark)/RTM-100, manufactured by Toyo Baldwin Co., Ltd.) is used to perform a test 5 times or more with different specimens having a measurement length of 50 mm at a tensile speed of 50 mm/min and to obtain an average value of breaking tenacities thereof. By dividing the obtained average value of breaking tenacities by a cross-sectional area of the membrane, a breaking strength S of the membrane is obtained.

Fig. 4 shows a relationship between a breaking strength So of the sample Mo before being immersed in the Fenton's reagent and a breaking strength St of the sample Mt after being immersed for t [h] in the Fenton's reagent as obtained by the above method. Using the obtained St and So, a membrane strength initial value ratio at the immersion time t [h] in the Fenton's reagent is defined as St/So.

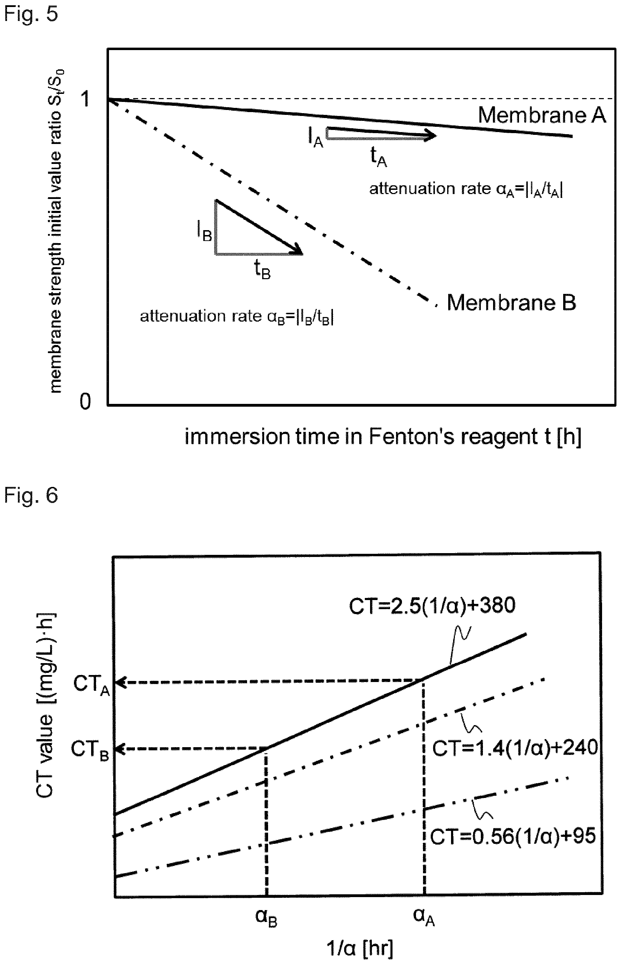

FIG. 5 shows a relationship between the membrane strength initial value ratio St/So obtained by the above method and the immersion time t [h] in the Fenton's reagent. When the time t [h] for which the separation membrane is immersed in the Fenton's reagent is prolonged, oxidative deterioration of the separation membrane is accelerated, and thus the membrane strength initial value ratio St/So is decreased. The relationship of St/So with respect to t [h] varies depending on the oxidation resistance of the separation membrane, and, in a membrane having an excellent oxidation resistance, an inclination of change in St/So with respect to t [h] becomes relatively small. For example, in FIG. 5, a membrane A has a small inclination of change in St/So with respect to t [h] as compared with a membrane B, that is, the membrane A has an excellent oxidation resistance as compared with the membrane B.

In the above accelerated oxidation test, when an absolute value of the inclination of the membrane strength initial value ratio St/So with respect to the time t [h] for which the separation membrane is immersed in the Fenton's reagent is expressed as an attenuation rate $\alpha$ [1/h], $\alpha$ varies depending on material, structure, and deterioration state of the membrane, and is an index representing an oxidation resistance of the membrane. In order to retain a life time of the separation membrane, the CT value is selected within a range that satisfies Expression (6) with respect to the attenuation rate $\alpha$ [1/h] of the separation membrane used. Furthermore, it is preferable to select the CT value within a range that satisfies Expression (7), and it is more preferable to select the CT value within a range that satisfies Expression (8).

$$CT \leq 2.5\,(1/\alpha) + 380 \qquad \text{Expression (6)}$$

$$CT \leq 1.4\,(1/\alpha) + 240 \qquad \text{Expression (7)}$$

$$CT \leq 0.56\,(1/\alpha) + 95 \qquad \text{Expression (8)}$$

FIG. 6 shows a relationship between $\alpha$ and the CT value as represented by Expression (6). By performing the chemical solution washing at a CT value that satisfies the expression (6), the filtration can be repeatedly performed while retaining a life time of the separation membrane. Here, "while retaining a life time of the separation membrane" means that a CT value given by the chemical solution washing is equal to or less than 1/250 of a CT value at which a membrane strength reaches 80% of an initial value ratio. In addition, more preferably, by performing the chemical solution washing at a CT value that satisfies the expression (7), the filtration can be repeatedly performed while retaining a life time of the separation membrane for a longer time. Even more preferably, by performing the chemical solution washing at a CT value that satisfies the expression (8), the filtration can be repeatedly performed while retaining a life time of the separation membrane for an even longer time.

Accordingly, by performing the chemical solution washing at a CT value that satisfies Expressions (4) and (6), it is possible to retain a life time of the separation membrane and to obtain a sufficient washing effect. That is the CT value is selected within a range that satisfies Expression (1). A lower limit of the CT value of Expression (1) is preferably selected within a range which has been replaced by Expression (5). An upper limit of the CT value of Expression (1) is preferably selected within a range which has been replaced by Expression (7), and more preferably selected within a range which has been replaced by Expression (8).

$$9.2 \times 10^{5}\text{F} - 1400 \leq CT \leq 2.5\,(1/\alpha) + 380 \qquad \text{Expression (1)}$$

The CT value is calculated from all washing steps involving hypochlorite, among washing steps performed in the step c. A specific calculation method of the CT value is described below.

In the step c, in a case where a step of bringing into contact with a chemical solution containing hypochlorite is performed, and immediately thereafter, the step proceeds to the step d, it is considered that, in the step c, a washing step with a chemical solution containing hypochlorite is performed once. In this case, a CT value in the once-performed washing step is set as a CT value in the step c.

On the other hand, in the step c, a step of bringing into contact with a chemical solution containing hypochlorite and a step of bringing into contact with a chemical solution without hypochlorite are performed several times in an alternate manner, and thereafter, the step proceeds to the step d. In this case, CT values are calculated for the respective steps using a chemical solution containing hypochlorite, and a total value thereof is set as a CT value in the step c.

In addition, in a case where a chemical solution used for the chemical solution washing contains a nonionic surfactant, a concentration of the nonionic surfactant is 0.05% by mass to 3% by mass, and preferably 0.1% by mass to 1% by mass. In addition, the concentration of the nonionic surfactant has a different critical micelle concentration depending on a type of the surfactant. However, in order to allow a sufficient washing effect to be exerted, the concentration is preferably set to be equal to or greater than the critical micelle concentration.

The pH of the chemical solution is 10 to 14, and preferably 11 to 13. Fouling substances in a microbial culture solution are mainly organic substances, and a washing power is increased at a high pH condition. When the pH is less than 10, a washing effect may be insufficient.

The temperature of the chemical solution is preferably 20°C to 50°C, and more preferably 30°C to 40°C. In a case where the temperature of the chemical solution is less than 20°C, a sufficient washing effect may not be exerted. On the other hand, in a case where the temperature of the chemical solution exceeds 50°C, decomposition of hypochlorite is accelerated, which is not preferable.

In the chemical solution washing, the chemical solution prepared in the chemical solution tank 10 is supplied to the membrane module 2, and is brought into contact with the separation membrane and an inside of the membrane module, so that washing is performed. An example of a washing method is, for example, an immersion washing in which the chemical solution is supplied to a raw liquid side flow path of the membrane module 2, and, once the inside of the membrane module is filled with the chemical solution, supply of the chemical solution is stopped and such a state is retained as it is.

As a method for further increasing a washing effect, there is a circulation washing in which the chemical solution is circulated between the chemical solution tank 10 and the raw liquid side flow path of the membrane module 2. By circulating the chemical solution, a sufficient amount of effective chlorine can be supplied to the membrane module.

As a method for further increasing a washing effect, there is a method of performing cross-flow filtration with the chemical solution. By performing the cross-flow filtration with the chemical solution, the chemical solution is allowed to even flow into micropores of the separation membrane. Thus, it can be expected to increase a washing effect. There are no particular limitations on a membrane surface linear velocity and a filtration flux at the time of performing the cross-flow filtration, and the cross-flow filtration may be performed under the same conditions as in the step a.

(Step d)

<Water Rinsing>

In the step d, water rinsing of the membrane module 2 with water is performed, which is aimed at washing the chemical solution remaining after the chemical solution washing in the step c. For a method for water rinsing, the same method as in the step b may be performed.

<Membrane Module>

A type of the membrane module used in the present invention is not particularly limited, and a flat membrane module, or a hollow-fiber membrane module can be used. Due to a large specific surface area and a large amount of liquid that can be filtrated per unit time, the hollow-fiber membrane is generally advantageous as compared with the flat membrane.

A structure of the separation membrane includes a composite membrane having a symmetric membrane having a uniform pore diameter as a whole or an asymmetric membrane of which pore diameters change in a thickness direction of the membrane, a composite membrane having a support layer for retaining strength and a separation-function layer for separating target substances.

An average pore diameter of the separation membrane may be appropriately selected depending on targets to be separated, and is preferably 0.01 $\mu$m to 1.0 $\mu$m for a purpose of separating microorganisms such as bacteria and fungi. When the average pore diameter is less than 0.01 $\mu$m, a water permeability is low, whereas when the average pore diameter exceeds 1.0 $\mu$m, there is a possibility that microorganisms leak.

In addition, in particular, in a case of carrying out filtration of beer, from the viewpoint of effect on flavor components and prevention of fouling, it is preferable to use a separation membrane having an average pore diameter of 0.3 $\mu$m to 1.0 $\mu$m, and it is more preferable to use a separation membrane having an average pore diameter of 0.4 $\mu$m to 0.8 $\mu$m. When the average pore diameter of the separation membrane is equal to or less than 0.3 $\mu$m, necessary flavor components are easily blocked and fouling also easily progresses.

A material of the separation membrane is not particularly limited, and the separation membrane can, for example, contain a fluorine-based resin such as polytetrafluoroethylene, polyvinylidene fluoride, polyvinyl fluoride, a tetrafluoroethylene/hexafluoropropylene copolymer, an ethylene/tetrafluoride copolymer, a cellulose ester such as cellulose acetate, cellulose acetate propionate, and cellulose acetate butyrate, a polysulfone-based resin such as polysulfone and polyethersulfone, or a resin such as polyacrylonitrile, polyimide, and polypropylene.

In particular, due to high heat resistance, physical strength, and chemical durability, a separation membrane formed of the fluorine-based resin or the polysulfone-based resin can be suitably used for filtration of a microbial culture solution.

In addition, besides the fluorine-based resin and the polysulfone-based resin, the separation membrane may further contain a hydrophilic resin. A hydrophilic property of the separation membrane is increased by the hydrophilic resin, which allows the membrane to have an improved water permeability. It is sufficient that the hydrophilic resin is any resin which can impart hydrophilicity to the separation membrane, and the hydrophilic resin is not limited to specific compounds. For example, cellulose ester, fatty acid vinyl ester, vinyl pyrrolidone, ethylene oxide, propylene oxide, polymethacrylic acid ester-based resin, and polyacrylic acid ester-based resin are suitably used.

Besides, any materials, shapes, dimensions, numerical values, forms, numbers, and disposed locations of the respective constituent elements in the above-described embodiments are used as long as the present invention can be achieved, and there are no limitations thereon.

EXAMPLES

<Measurement of average pore diameter of separation membrane>

The average pore diameter of the separation membrane was measured by the following method.

A raw liquid obtained by dispersing Uniform Latex particles manufactured by Seradyn having particle diameters of 0.103 $\mu$m, 0.200 $\mu$m, 0.304 $\mu$m, 0.434 $\mu$m, 0.580 $\mu$m, 0.774 $\mu$m, 0.862 $\mu$m, 1.000 $\mu$m, and 1.104 $\mu$m in pure water to a concentration of 20 mg/L was used as supplying water, and cross-flow filtration was performed with a supplying pressure of 3 kPa and a membrane surface linear velocity of 0.2 m/s on average being given, to obtain filtrated water. Polystyrene latex concentrations in the supplying water and the filtrated water were obtained from an ultraviolet-visible spectrophotometer (UV 2450, manufactured by Shimadzu Corporation), and a blocking ratio was obtained from the following expression. A filtrated water concentration was obtained by sampling a liquid 30 minutes after the start of filtration.

$$\text{Blocking ratio } [\%] = (1 - \text{filtrated water concentration } [\text{mg/L}]/\text{supplying water}$$

$$\text{concentration } [\text{mg/L}]) \times 100$$

From an approximate curve of the particle diameters of such Uniform Latex particles and the blocking ratio, a particle diameter at a blocking ratio of 90% was calculated and this was set as the average pore diameter of the separation membrane.

<Measurement of water permeability restoration rate>

A water permeability restoration rate after the chemical solution washing was obtained from a proportion of a pure water permeability coefficient after the chemical solution washing with respect to a pure water permeability coefficient of an unused membrane module. The pure water permeability coefficient was obtained from the following expression by supplying pure water at 25°C into the membrane module at a pressure of 50 kPa and measuring a permeated water amount of the pure water that has permeated through a membrane.

$$\text{Water permeability restoration rate } [\%] = \text{pure water permeability coefficient after}$$

$$\text{chemical solution washing } [\text{m}^3/\text{m}^2/\text{hr}]/\text{pure water permeability coefficient in a new module}$$

$$\text{(unused) } [\text{m}^3/\text{m}^2/\text{hr}] \times 100$$

$$\text{Pure water permeability coefficient } [\text{m}^3/\text{m}^2/\text{hr}] = \text{permeated water amount}$$

$$[\text{m}^3]/(\text{membrane area } [\text{m}^2] \times \text{measurement time } [\text{hr}])$$

<Measurement of total organic carbon amount>

A specimen solution was filtrated with a syringe filter (manufactured by Advantec Corporation, pore diameter of 0.45 $\mu$m, material of PTFE), and, from the obtained filtrate, an amount of non-purgeable organic carbon (NPOC) was measured using a TOC-VCSH TOC meter (manufactured by Shimadzu Corporation).

<Measurement of total sugar concentration>

Total sugars in a specimen solution were quantified by a phenol-sulfuric acid method. That is, the specimen and a 5% phenol solution were mixed in equal amounts, and then concentrated sulfuric acid in an amount of 1.5 times the mixture was quickly added and stirred. The mixture was kept at a constant temperature (20°C to 30°C) for 20 to 30 minutes. Thereafter, an absorbance of the mixed solution at a wavelength of 480 nm was measured using an ultraviolet-visible spectrophotometer (UV 2450, manufactured by Shimadzu Corporation). Meanwhile, a calibration curve was prepared with a glucose standard solution, and the obtained calibration curve was used to calculate a total sugar concentration (conversion value in terms of glucose) in the specimen solution.

<Measurement of protein concentration>

1350 $\mu$L of Coomassie (Bradford) Protein Assay Reagent (manufactured by Thermo Fisher Scientific) as a Bradford reagent was mixed in 150 $\mu$L of the specimen solution, and left to stand at room temperature for 10 minutes for reaction. After the reaction, an absorbance of the mixed solution at a wavelength of 470 nm was measured using an ultraviolet-visible spectrophotometer (UV 2450, manufactured by Shimadzu Corporation). Meanwhile, a calibration curve was prepared by reacting a bovine serum albumin standard specimen and the Bradford reagent in the same manner as above, and the obtained calibration curve was used to calculate a protein concentration in the specimen solution.

<Accelerated oxidation test using Fenton's reagent>

A Fenton's reagent was prepared by adding iron sulfate (II) heptahydrate (manufactured by Wako Pure Chemical Industries, Ltd.) to hydrogen peroxide (manufactured by Wako Pure Chemical Industries, Ltd.). At this time, adjustment was made so that an $H_2O_2$ concentration was 5,000 ppm and a $Fe^{2+}$ concentration was 300 ppm. In addition, separation membranes were immersed in ethanol to cause hydrophilization. The hydrophilized separation membranes were immersed in the Fenton's reagent which had been adjusted to the above-mentioned concentrations for 25 hours, 50 hours, 100 hours, 150 hours, and 250 hours, respectively. The separation membranes immersed in the Fenton's reagent for the respective period of times were washed with distilled water and vacuum dried at room temperature.

<Calculation of attenuation rate $\alpha$>

Membrane strengths for the separation membranes before being subjected to the accelerated oxidation test and the separation membranes after the respective immersion times obtained in the accelerated oxidation test were measured, respectively. A measurement method will be described later.
A membrane strength initial value ratio St/So, which is a ratio of a membrane strength of a separation membrane after being immersed in the Fenton's reagent with respect to a membrane strength of the separation membrane before being subjected to the accelerated oxidation test was calculated for the separation membranes after the respective immersion times. The membrane strength initial value ratio St/So with respect to time [h] for which the separation membrane is immersed in the Fenton's reagent was plotted to a graph and an absolute value of an inclination thereof was calculated as the attenuation rate $\alpha$ [1/h].

<Measurement of membrane strength>

A tensile test machine (TENSILON (registered trademark)/RTM-100, manufactured by Toyo Baldwin Co., Ltd.) was used to perform a test 5 times or more with different specimens having a measurement length of 50 mm at a tensile speed of 50 mm/min and to obtain an average value of breaking tenacities thereof. The obtained average value of breaking tenacities was divided by a cross-sectional area of the membrane to obtain a breaking strength of the membrane.

(Reference Example 1)

38 parts by mass of a vinylidene fluoride homopolymer having a weight average molecular weight of 417,000 and 62 parts by mass of $\gamma$-butyrolactone were mixed and dissolved at 160°C. This polymer solution was discharged from a double tube spinneret while allowing a 85% by mass $\gamma$-butyrolactone aqueous solution as a hollow portion-forming liquid to be accompanied thereby, and was coagulated in a cooling bath, which is provided 30 mm below the spinneret and contains a 85% by mass $\gamma$-butyrolactone aqueous solution having a temperature of 10°C, to prepare a hollow-fiber membrane having a sphere-like structure. The obtained hollow-fiber membrane had an outer diameter of 1,250 $\mu$m, an inner diameter of 800 $\mu$m, and an average pore diameter of 0.5 $\mu$m.

(Reference Example 2)

38 parts by mass of a vinylidene fluoride homopolymer having a weight average molecular weight of 417,000 and 62 parts by mass of $\gamma$-butyrolactone were mixed and dissolved at 160°C. This polymer solution was discharged from a double tube spinneret while allowing a 85% by mass $\gamma$-butyrolactone aqueous solution as a hollow portion-forming liquid to be accompanied thereby, and was coagulated in a cooling bath, which is provided 30 mm below the spinneret and contains a 85% by mass $\gamma$-butyrolactone aqueous solution having a temperature of 5°C, to prepare a hollow-fiber membrane having a sphere-like structure. The obtained hollow-fiber membrane had an outer diameter of 1250 $\mu$m, an

inner diameter of 800 $\mu$m, and an average pore diameter of 0.3 $\mu$m.

(Reference Example 3)

38 parts by mass of a vinylidene fluoride homopolymer having a weight average molecular weight of 417,000 and 62 parts by mass of $\gamma$-butyrolactone were mixed and dissolved at 160°C. This polymer solution was discharged from a double tube spinneret while allowing a 85% by mass $\gamma$-butyrolactone aqueous solution as a hollow portion-forming liquid to be accompanied thereby, and was coagulated in a cooling bath, which is provided 30 mm below the spinneret and contains a 85% by mass $\gamma$-butyrolactone aqueous solution having a temperature of 25°C, to prepare a hollow-fiber membrane having a sphere-like structure. The obtained hollow-fiber membrane had an outer diameter of 1250 $\mu$m, an inner diameter of 800 $\mu$m, and an average pore diameter of 1.0 $\mu$m.

(Reference Example 4)

Millipore Express Plus (manufactured by Millipore Corporation) which is a commercially available flat membrane formed of polyethersulfone was used. An average pore diameter thereof was 0.45 $\mu$m.

(Example 1)

Using a hollow-fiber membrane module prepared using the hollow-fiber membrane of Reference Example 1, cross-flow filtration of beer was performed with a filtration apparatus of FIG. 1 (step a). For the beer, Fujizakura Heights Beer Pils (manufactured by Fuji Kanko Kaihatsu Co., Ltd.) which is a commercially available unfiltrated beer was used. The above beer is denoted by beer A. In the cross-flow filtration, a membrane surface linear velocity was 0.5 m/s, a filtration flux was 1 $m^3/m^2/d$, a backwashing flux was 2 $m^3/m^2/d$, a filtration time per cycle was 29 minutes, and a backwashing time was 1 minute per cycle, under which filtration and backwashing were repeated.

Filtration of the beer and backwashing were repeated. When R/Ro reached 10, the filtration was stopped and drainage was performed, and rinsing with pure water was performed (step b). The rinsing with pure water was performed for 10 minutes at a membrane surface linear velocity of 0.5 m/s and a filtration flux of 1 $m^3/m^2/d$, and, thereafter, backwashing was performed at 2 $m^3/m^2/d$ for 3 minutes.

Subsequently, chemical solution washing of the hollow-fiber membrane module was performed (step c). A chemical solution which contains sodium hypochlorite (having an effective chlorine concentration of 0.1% by mass) and a nonionic surfactant Tween 20 (manufactured by Wako Pure Chemical Industries, Ltd., polyoxyethylene sorbitan monolaurate, HLB of 16.7, concentration of 0.2% by mass) and which had been adjusted to pH 12 with sodium hydroxide was used. Temperature of the chemical solution was 35°C. This chemical solution was circulated in the membrane module for 2 hours to perform washing. At this time, a membrane surface linear velocity was 0.5 m/s and a filtration flux was 1 $m^3/m^2/d$.

Drainage was performed for the chemical solution and subsequently rinsing with pure water was performed (step d). The rinsing with pure water was performed at a membrane surface linear velocity of 0.5 m/s and a filtration flux of 1 $m^3/m^2/d$ for 5 minutes. Thereafter, drainage was performed, rinsing with pure water was performed again, and the same operation was repeated five times in total.

After repeating the above steps a to d three times, a pure water permeability coefficient of the membrane module was measured and a water permeability restoration rate after the chemical solution washing was calculated. As a result, a restoration rate was 81%.

(Examples 2, 4, 6, Examples 10 to 12 and 14 to 21, Example 23, Illustrative Examples 3, 5, 9 and 13[1], and Comparative Examples 3 to 8)

Filtration of a target liquid was performed in the same manner as in Example 1 except that conditions were changed to those described in Tables 1 to 5. In a case where the chemical solution washing was performed twice, a first-round washing was performed and then a second-round washing was performed. A membrane surface linear velocity and a filtration flux at the time of performing the second-round washing were the same as the first-round washing.

For a raw liquid for filtration, Fujizakura Heights Beer Pils (manufactured by Fuji Kanko Kaihatsu Co., Ltd.) as beer A or Ginga Kogen Beer Pilsner(manufactured by Ginga Kogen Beer Co., Ltd.) as beer B was used.

As the nonionic surfactant, Tween 20 (polyoxyethylene sorbitan monolaurate, HLB of 16.7, concentration of 0.2% by mass, manufactured by Wako Pure Chemical Industries, Ltd.), PERSOFT NK-60 (manufactured by NOF Corporation, polyoxyethylene alkyl ether, HLB of 12.0), Triton X-100 (manufactured by Wako Pure Chemical Industries, Ltd., polyoxyethylene octylphenyl ether, HLB of 13.5), or NONION K-230 (manufactured by NOF Corporation, polyoxyethylene lauryl ether, HLB of 17.5) was used.

As the anionic surfactant, SDS (manufactured by Wako Pure Chemical Industries, Ltd., sodium dodecyl sulfate) or NISSAN TRAX K-40 (manufactured by NOF Corporation, polyoxyethylene lauryl ether sulfuric acid ester sodium salt) was used.

After completion of the filtration, a pure water permeability coefficient of the membrane module was measured to calculate a water permeability restoration rate after the chemical solution washing. The results are shown all together in Tables 1 to 5.

(Example 7 and Illustrative Example 8)

Using a flat membrane module prepared using the flat membrane of Reference Example 4, cross-flow filtration of beer was performed with a filtration apparatus of FIG. 1 (step a). Filtration of a target liquid was carried out in the same manner as in Example 1 except that conditions were changed to those described in Table 1 for a subsequent filtration operation method.

(Example 22)

Budding yeast (Saccharomyces cerevisiae CM 3260 strain) was cultured at 30°C for 24 hours in a liquid medium containing 20 g/l of glucose, 5 g/l of ammonium sulfate, 0.59 g/l of potassium chloride, 0.1 g/l of sodium chloride, 0.1 g/l of calcium chloride, 0.5 g/l of magnesium sulfate heptahydrate, 0.02 g/l of uracil, 0.06 g/l of leucine, 0.02 g/l of histidine, and 0.04 g/l of tryptophan. For this yeast culture solution, filtration and chemical solution washing of the membrane module were repeated three times in the same manner as in Example 1.

A water permeability restoration rate after chemical solution washing was calculated, and, as a result, a restoration rate was 90%.

(Comparative Example 1)

For an activated sludge dispersion solution of which a seed sludge is an activated sludge acquired from a wastewater treatment facility of a rural village and is acclimatized with sewage, filtration of a target liquid was carried out in the same manner as in Example 1 except that conditions were changed to those described in Table 5. In the chemical solution washing, only hypochlorite was used.

A water permeability restoration rate after the chemical solution washing was calculated, and, as a result, a restoration rate was 83%. In a case of a separation membrane which was used to filtrate an activated sludge with low total sugar concentration and protein concentration and was closed, a high water permeability restoration rate was obtained with chemical solution washing using only hypochlorite.

(Comparative Example 2)

E. coli was cultured at 30°C for 24 hours in a liquid medium containing 20 g/l of LB Broth Base (manufactured by Invitrogen). For this E. coli culture solution, filtration of a target liquid was carried out in the same manner as in Example 1 except that conditions were changed to those described in Table 5. In the chemical solution washing, only hypochlorite was used.

A water permeability restoration rate after the chemical solution washing was calculated, and, as a result, a restoration rate was 81%. In a case of a separation membrane which was used to filtrate the E. coli culture solution with low total sugar concentration and protein concentration and was closed, a high water permeability restoration rate was obtained with chemical solution washing using only hypochlorite.

Table 1

| | Separation membrane | | | Permeation liquid | | | | Membrane surface linear velocity [m/s] | Filtration flux [m3/m2/d] | Back-washing flux [m3/m2/d] | Filtration time [min] | Back-washing time [min] | Proceeding condition for chemical solution washing R/R0 | Water rinsing before chemical solution washing |
|---|---|---|---|---|---|---|---|---|---|---|---|---|---|---|
| | Membrane pore diameter [μm] | Membrane material | $\alpha$ [1/h] | Type | Total sugar concentration [mg/L] | Protein concentration [mg/L] | F value | | | | | | | |
| Ex. 1 / Ref Ex. 1 | 0.5 | PVDF | $5.0 \times 10^{-5}$ | Beer A | 43,000 | 180 | 0.0026 | 0.5 | 1 | 2 | 29 | 1 | 10 | Yes |
| Ex. 2 / Ref Ex. 1 | 0.5 | PVDF | $5.0 \times 10^{-5}$ | Beer A | 43,000 | 180 | 0.0026 | 0.5 | 1 | 2 | 29 | 1 | 10 | Yes |
| Ill. Ex. 3 / Ref Ex. 1 | 0.5 | PVDF | $5.0 \times 10^{-5}$ | Beer A | 43,000 | 180 | 0.0026 | 0.5 | 1 | 2 | 29 | 1 | 10 | Yes |
| Ex. 4 / Ref Ex. 1 | 0.5 | PVDF | $5.0 \times 10^{-5}$ | Beer B | 26,000 | 340 | 0.0050 | 0.5 | 1 | 2 | 29 | 1 | 10 | Yes |
| Ill. Ex. 5 / Ref Ex. 1 | 0.5 | PVDF | $5.0 \times 10^{-5}$ | Beer B | 26,000 | 340 | 0.0050 | 0.5 | 1 | 2 | 29 | 1 | 10 | Yes |
| Ex. 6 / Ref Ex. 1 | 0.5 | PVDF | $5.0 \times 10^{-5}$ | Beer B | 26,000 | 340 | 0.0050 | 0.5 | 1 | 2 | 29 | 1 | 10 | Yes |

Table 1 (Continued)

| | Chemical solution used First round | | | | Chemical solution used Second round | | | | Hypochlorite CT value [(mg/L)·h] | Surfactant HLB | Water permeability restoration rate after chemical solution washing [%] |
|---|---|---|---|---|---|---|---|---|---|---|---|
| | Composition | pH | Contact time [h] | Temperature | Composition | pH | Contact time [h] | Temperature | | | |
| Ex. 1 | Sodium hypochlorite 0.1% Tween 20 0.2% | 12 | 2 | 35°C | - | | | | 2,000 | 16.7 | 81 |
| Ex. 2 | Sodium hypochlorite 0.3% Tween 20 0.2% | 12 | 2 | 35°C | - | | | | 6,000 | 16.7 | 88 |
| Illustr. Ex. 3 | Sodium hypochlorite 0.01% Tween 20 0.2% | 12 | 2 | 35°C | - | | | | 200 | 16.7 | 60 |
| Ex. 4 | Sodium hypochlorite 0.2% Tween 20 0.2% | 12 | 2 | 35°C | - | | | | 4,000 | 16.7 | 81 |
| Illustr. Ex. 5 | Sodium hypochlorite 0.1% Tween 20 0.2% | 12 | 2 | 35°C | - | | | | 2,000 | 16.7 | 75 |
| Ex. 6 | Sodium hypochlorite 0.4% Tween 20 0.2% | 12 | 1 | 35°C | - | | | | 4,000 | 16.7 | 84 |

Table 2

| | Separation membrane | | | Permeation liquid | | | | Membrane surface linear velocity [m/s] | Filtration flux [m3/m2/d] | Back-washing flux [m3/m2/d] | Filtration time [min] | Back-washing time [min] | Proceeding condition for chemical solution washing R/R0 | Water rinsing before chemical solution washing |
|---|---|---|---|---|---|---|---|---|---|---|---|---|---|---|
| | Membrane pore diameter [μm] | Membrane material | $\alpha$ [1/h] | Type | Total sugar concentration [mg/L] | Protein concentration [mg/L] | F value | | | | | | | |
| Ex. 7 / Ref Ex. 4 | 0.45 | PES | $5.3 \times 10^{-4}$ | Beer A | 43,000 | 180 | 0.0026 | 0.5 | 2 | 2 | 29 | 1 | 10 | Yes |
| Ill. Ex. 8 / Ref Ex. 4 | 0.45 | PES | $5.3 \times 10^{-4}$ | Beer A | 43,000 | 180 | 0.0026 | 0.5 | 2 | 2 | 29 | 1 | 10 | Yes |
| Ill. Ex. 9 / Ref Ex. 1 | 0.5 | PVDF | $5.0 \times 10^{-5}$ | Beer A | 43,000 | 180 | 0.0026 | 0.5 | 1 | 2 | 29 | 1 | 28 | Yes |
| Ex. 10 / Ref Ex. 1 | 0.5 | PVDF | $5.0 \times 10^{-5}$ | Beer A | 43,000 | 180 | 0.0026 | 0.5 | 1 | - | 30 | - | 10 | Yes |
| Ex. 11 / Ref Ex. 1 | 0.5 | PVDF | $5.0 \times 10^{-5}$ | Beer A | 43,000 | 180 | 0.0026 | 0.5 | 1 | 2 | 29 | 1 | 16 | Yes |
| Ex. 12 / Ref Ex. 1 | 0.5 | PVPF | $5.0 \times 10^{-5}$ | Beer A | 43,000 | 180 | 0.0026 | 3 | 2 | 3 | 19 | 1 | 5 | Yes |

Table 2 (Continued)

| | Composition | pH | Contact time [h] | Temperature | Composition | pH | Contact time [h] | Temperature | Hypochlorite CT value [(mg/L)·h] | Surfactant HLB | Water permeability restoration rate after chemical solution washing [%] |
|---|---|---|---|---|---|---|---|---|---|---|---|
| | Chemical solution used First round | | | | Chemical solution used Second round | | | | | | |
| Ex. 7 | Sodium hypochlorite 0.05% Tween 20 0.2% | 12 | 2 | 35°C | | | - | | 1,000 | 16.7 | 80 |
| Illustr. Ex. 8 | Sodium hypochlorite 0.01% Tween 20 0.2% | 12 | 2 | 35°C | | | - | | 200 | 16.7 | 71 |
| Illustr. Ex. 9 | Sodium hypochlorite 0.3% Tween 20 0.2% | 12 | 2 | 35°C | | | - | | 6,000 | 16.7 | 70 |
| Ex. 10 | Sodium hypochlorite 0.3% Tween 20 0.2% | 12 | 2 | 35°C | | | - | | 6,000 | 16.7 | 78 |
| Ex. 11 | Sodium hypochlorite 0.3% Tween 20 0.2% | 12 | 2 | 35°C | | | - | | 6,000 | 16.7 | 82 |
| Ex. 12 | Sodium hypochlorite 0.3% Tween 20 0.2% | 12 | 2 | 35°C | | | - | | 6,000 | 16.7 | 90 |

Table 3

| | | Membrane pore diameter [µm] | Membrane material | α [1/h] | Type | Total sugar concentration [mg/L] | Protein concentration [mg/L] | F value | Membrane surface linear velocity [m/s] | Filtration flux [m³/m²/d] | Back-washing flux [m³/m²/d] | Filtration time [min] | Back-washing time [min] | Proceeding condition for chemical solution washing R/R₀ | Water rinsing before chemical solution washing |
|---|---|---|---|---|---|---|---|---|---|---|---|---|---|---|---|
| | | Separation membrane | | | Permeation liquid | | | | | | | | | | |
| Ill. Ex. 13 | Ref Ex. 1 | 0.5 | PVDF | $5.0 \times 10^{-5}$ | Beer A | 43,000 | 180 | 0.0026 | 0.5 | 1 | 2 | 29 | 1 | 10 | Yes |
| Ex. 14 | Ref Ex. 1 | 0.5 | PVDF | $5.0 \times 10^{-5}$ | Beer A | 43,000 | 180 | 0.0026 | 0.5 | 1 | 2 | 29 | 1 | 10 | Yes |
| Ex. 15 | Ref Ex. 1 | 0.5 | PVDF | $5.0 \times 10^{-5}$ | Beer A | 43,000 | 180 | 0.0026 | 0.5 | 1 | 2 | 29 | 1 | 10 | Yes |
| Ex. 16 | Ref Ex. 1 | 0.5 | PVDF | $5.0 \times 10^{-5}$ | Beer A | 43,000 | 180 | 0.0026 | 0.5 | 1 | 2 | 29 | 1 | 10 | Yes |
| Ex. 17 | Ref Ex. 1 | 0.5 | PVDF | $5.0 \times 10^{-5}$ | Beer A | 43,000 | 180 | 0.0026 | 0.5 | 1 | 2 | 29 | 1 | 10 | Yes |
| Ex. 18 | Ref Ex. 1 | 0.5 | PVDF | $5.0 \times 10^{-5}$ | Beer A | 43,000 | 180 | 0.0026 | 0.5 | 1 | 2 | 29 | 1 | 10 | Yes |

Table 3 (continued)

| | Composition | pH | Contact time [h] | Temperature | Composition | pH | Contact time [h] | Temperature | Hypochlorite CT value [(mg/L)·h] | Surfactant HLB | Water permeability restoration rate after chemical solution washing [%] |
|---|---|---|---|---|---|---|---|---|---|---|---|
| | Chemical solution used First round | | | | Chemical solution used Second round | | | | | | |
| Illustr. Ex. 13 | Sodium hypochlorite 0.3% PERSOFT NK-60 0.2% | 12 | 2 | 35°C | | | - | | 6,000 | 12 | 72 |
| Ex. 14 | Sodium hypochlorite 0.3% Triton X-100 0.2% | 12 | 2 | 35°C | | | - | | 6,000 | 13.5 | 85 |
| Ex. 15 | Sodium hypochlorite 0.3% NONION K-230 0.2% | 12 | 2 | 35°C | | | - | | 6,000 | 17.5 | 78 |
| Ex. 16 | Sodium hypochlorite 0.05% Tween 20 0.05% | 12 | 2 | 35°C | | | - | | 1,000 | 16.7 | 76 |
| Ex. 17 | Sodium hypochlorite 0.3% Tween 20 0.2% | 9 | 2 | 35°C | | | - | | 6,000 | 16.7 | 73 |
| Ex. 18 | Sodium hypochlorite 0.3% Tween 20 0.2% | 10 | 2 | 35°C | | | - | | 6,000 | 16.7 | 80 |

Table 4

| | | Separation membrane | | | Permeation liquid | | | | Membrane surface linear velocity [m/s] | Filtration flux [m³/m²/d] | Back-washing flux [m³/m²/d] | Filtration time [min] | Back-washing time [min] | Proceeding condition for chemical solution washing R/R₀ | Water rinsing before chemical solution washing |
|---|---|---|---|---|---|---|---|---|---|---|---|---|---|---|---|
| | | Membrane pore diameter [μm] | Membrane material | α [1/h] | Type | Total sugar concentration [mg/L] | Protein concentration [mg/L] | F value | | | | | | | |
| Ex. 19 | Ref Ex. 1 | 0.5 | PVDF | $5.0 \times 10^{-5}$ | Beer A | 43,000 | 180 | 0.0026 | 0.5 | 1 | 2 | 29 | 1 | 10 | Yes |
| Ex. 20 | Ref Ex. 2 | 0.3 | PVDF | $4.3 \times 10^{-5}$ | Beer A | 43,000 | 180 | 0.0026 | 0.5 | 1 | 2 | 29 | 1 | 10 | Yes |
| Ex. 21 | Ref Ex. 3 | 1 | PVDF | $5.1 \times 10^{-5}$ | Beer A | 43,000 | 180 | 0.0026 | 0.5 | 1 | 2 | 29 | 1 | 10 | Yes |
| Ex. 22 | Ref Ex. 1 | 0.5 | PVDF | $5.0 \times 10^{-5}$ | Yeast culture solution | 18,000 | 95 | 0.0040 | 0.5 | 1 | 2 | 29 | 1 | 10 | Yes |
| Ex. 23 | Ref Ex. 1 | 0.5 | PVDF | $5.0 \times 10^{-5}$ | Beer A | 43,000 | 180 | 0.0026 | 0.5 | 1 | 2 | 29 | 1 | 10 | Yes |

Table 4 (continued)

| | Chemical solution used First round | | | | Chemical solution used Second round | | | | Hypochlorite CT value [(mg/L)·h] | Surfactant HLB | Water permeability restoration rate after chemical solution washing [%] |
|---|---|---|---|---|---|---|---|---|---|---|---|
| | Composition | pH | Contact time [h] | Temperature | Composition | pH | Contact time [h] | Temperature | | | |
| Ex. 19 | Sodium hypochlorite 0.3% Tween 20 0.2% | 12 | 2 | 20°C | - | | | | 6,000 | 16.7 | 80 |
| Ex. 20 | Sodium hypochlorite 0.3% Tween 20 0.2% | 12 | 2 | 35°C | - | | | | 6,000 | 16.7 | 84 |
| Ex. 21 | Sodium hypochlorite 0.3% Tween 20 0.2% | 12 | 2 | 35°C | - | | | | 6,000 | 16.7 | 88 |
| Ex. 22 | Sodium hypochlorite 0.3% Tween 20 0.2% | 12 | 2 | 35°C | - | | | | 6,000 | 16.7 | 90 |
| Ex. 23 | Sodium hypochlorite 0.3% | 12 | 2 | 35°C | Tween 20 0.2% | 12 | 2 | 35°C | 6,000 | - | 84 |

Table 5

| | | Separation membrane | | | Permeation liquid | | | | Membrane surface linear velocity [m/s] | Filtration flux [m³/m²/d] | Back-washing flux [m³/m²/d] | Filtration time [min] | Back-washing time [min] | Proceeding condition for chemical solution washing $R/R_0$ | Water rinsing before chemical solution washing |
| | | Membrane pore diameter [μm] | Membrane material | $\alpha$ [1/h] | Type | Total sugar concentration [mg/L] | Protein concentration [mg/L] | F value | | | | | | | |
|---|---|---|---|---|---|---|---|---|---|---|---|---|---|---|---|
| Comp Ex. 1 | Ref Ex. 1 | 0.5 | PVDF | $5.0 \times 10^{-5}$ | Activated sludge Treated water | 3.8 | 120 | 0.0005 | 0.5 | 2 | 2 | 29 | 1 | 10 | Yes |
| Comp Ex. 2 | Ref Ex. 1 | 0.5 | PVDF | $5.0 \times 10^{-5}$ | E. coli culture solution | 300 | 70 | 0.0061 | 0.5 | 2 | 2 | 29 | 1 | 10 | Yes |
| Comp Ex. 3 | Ref Ex. 1 | 0.5 | PVDF | $5.0 \times 10^{-5}$ | Beer A | 43,000 | 180 | 0.0026 | 0.5 | 1 | 2 | 29 | 1 | 10 | Yes |
| Comp Ex. 4 | Ref Ex. 1 | 0.5 | PVDF | $5.0 \times 10^{-5}$ | Beer A | 43,000 | 180 | 0.0026 | 0.5 | 1 | 2 | 29 | 1 | 10 | Yes |
| Comp Ex. 5 | Ref Ex. 1 | 0.5 | PVDF | $5.0 \times 10^{-5}$ | Beer A | 43,000 | 180 | 0.0026 | 0.5 | 1 | 2 | 29 | 1 | 10 | Yes |
| Comp Ex. 6 | Ref Ex. 1 | 0.5 | PVDF | $5.0 \times 10^{-5}$ | Beer A | 43,000 | 180 | 0.0026 | 0.5 | 1 | 2 | 29 | 1 | 10 | Yes |
| Comp Ex. 7 | Ref Ex. 1 | 0.5 | PVDF | $5.0 \times 10^{-5}$ | Beer A | 43,000 | 180 | 0.0026 | 0.5 | 1 | 2 | 29 | 1 | 10 | Yes |
| Comp Ex. 8 | Ref Ex. 1 | 0.5 | PVDF | $5.0 \times 10^{-5}$ | Beer A | 43,000 | 180 | 0.0026 | 0.5 | 1 | 2 | 29 | 1 | 10 | No |

Table 5 (continued)

| | Chemical solution used First round | | | | Chemical solution used Second round | | | | Hypochlorite CT value [(mg/L) · h] | Surfactant HLB | Water permeability restoration rate after chemical solution washing [%] |
|---|---|---|---|---|---|---|---|---|---|---|---|
| | Composition | pH | Contact time [h] | Temperature | Composition | pH | Contact time [h] | Temperature | | | |
| Comp Ex. 1 | Sodium hypochlorite 0.3% | 12 | 2 | 35°C | - | | | | 6,000 | - | 83 |
| Comp Ex. 2 | Sodium hypochlorite 0.3% | 12 | 2 | 35°C | - | | | | 6,000 | - | 81 |
| Comp Ex. 3 | NaOH 0.01 N | 12 | 2 | 35°C | - | | | | - | - | 28 |
| Comp Ex. 4 | Tween 20 0.2% | 12 | 2 | 35°C | - | | | | - | 16.7 | 40 |
| Comp Ex. 5 | Sodium hypochlorite 0.3% | 12 | 2 | 35°C | - | | | | 6,000 | - | 58 |
| Comp Ex. 6 | Sodium hypochlorite 0.3% SDS 0.2% | 12 | 2 | 35°C | - | | | | 6,000 | - (Anionic) | 59 |
| Comp Ex. 7 | Sodium hypochlorite 0.3% NISSAN TRAX K-40 0.2% | 12 | 2 | 35°C | - | | | | 6,000 | - (Anionic) | 59 |
| Comp Ex. 8 | Sodium hypochlorite 0.05% Tween 20 0.2% | 12 | 2 | 35°C | - | | | | 1,000 | 16.7 | 62 |

EP 3 438 243 B1

In Comparative Examples 3 to 8 in which high total sugar concentration and protein concentration are exhibited in a filtrate that has permeated through the separation membrane, a water permeability restoration rate after the chemical solution washing was 28% to 62%. On the contrary, in all of Examples 1, 2, 4, 6, 7, 10 to 12 and 14 to 23, the water permeability restoration rate was equal to or greater than 60%. From these results, it has been found that the filtration method of the present invention enables stable filtration of a microbial culture solution over a long period of time.

INDUSTRIAL APPLICABILITY

The method for filtrating a microbial culture solution of the present invention can be used for filtration of a microbial culture solution in a field of fermentation industry, and a field of food industry.

DESCRIPTION OF REFERENCE NUMERALS AND SIGNS

1:      Raw liquid tank
2:      Membrane module
3:      Raw liquid pump
4:      Circulation control valve
5:      Filtration control valve
6:      Filtrate tank
7:      Backwashing liquid tank
8:      Backwashing pump
9:      Backwashing control valve
10:     Chemical solution tank
11      to 20: Valves
21      to 23: Pressure gauges
24      to 26: Flow meters

Claims

1.   A method for filtrating a microbial culture solution using a membrane module, the method comprising:

(a) a step of performing cross-flow filtration of the microbial culture solution using the membrane module;
(b) a step of performing water rinsing of the membrane module after the step a;
(c) a step of bringing the membrane module into contact with a chemical solution after the step b; and
(d) a step of performing water rinsing of the membrane module after the step c,

wherein a filtrate that has permeated through the membrane module by the cross-flow filtration in the step a has a total sugar concentration of 1,000 mg/L to 100,000 mg/L, determined according to the description and a protein concentration of 50 mg/L to 1,000 mg/L, determined according to the description,

the step c includes a step (c-1) of bringing the membrane module into contact with a chemical solution containing hypochlorite and a nonionic surfactant, and
the steps a to d are repeated in this order, and
wherein, in the cross-flow filtration in the step a, a membrane surface linear velocity of the microbial culture solution is set to be 0.1 m/s to 3.0 m/s and a filtration flux is set to be 0.1 m$^3$/m$^2$/d to 2.0 m$^3$/m$^2$/d,
wherein the hypochlorite has an effective chlorine concentration of 0.05% by mass to 1% by mass, and the nonionic surfactant has a nonionic surfactant concentration of 0.05% by mass to 3% by mass and a hydrophilic-lipophilic balance (HLB) of 13 to 17,
wherein the chemical solution has a pH of 10 to 14, and
wherein the step c has a step which is operated within a range where a product CT [(mg/L) · h] of a hypochlorite concentration C [mg/L] in the chemical solution and a contact time T [h] between the hypochlorite and a separation membrane contained in the membrane module satisfies Expression (1):

$$9.2 \times 10^5 F - 1400 \leq CT \leq 2.5\,(1/\alpha) + 380 \quad \text{Expression (1)}$$

in Expression (1), F represents a ratio of the protein concentration [mg/L] in the filtrate which has permeated through the membrane module by the cross-flow filtration in the step a with respect to a total organic carbon amount [mg/L] in the filtrate, and $\alpha$ represents an absolute value [1/h] of an attenuation rate of a membrane strength initial value ratio with respect to an immersion time in a case where the separation membrane is immersed in a Fenton's reagent which is a mixed solution of 5,000 ppm of $H_2O_2$ and 300 ppm of $Fe^{2+}$, the total organic carbon amount and the attenuation rate $\alpha$ being determined according to the description.

2. The method for filtrating a microbial culture solution using a membrane module according to claim 1,

wherein in a case where Expression (2) is satisfied in the step a, the step proceeds to the step b:

$$5 \leq R/R_0 \leq 16 \quad \text{Expression (2)}$$

in Expression (2), R represents a filtration resistance value [$m^{-1}$] during performing the cross-flow filtration of the microbial culture solution in the step a, and Ro represents a filtration resistance value [$m^{-1}$] at a start of the cross-flow filtration.

3. The method for filtrating a microbial culture solution using a membrane module according to claim 1 or 2,

wherein the step a comprises:

(a-1) a step of performing cross-flow filtration of the microbial culture solution using the membrane module; and
(a-2) a step of performing backwashing of the membrane module,

when the cross-flow filtration is performed, a membrane surface linear velocity of the microbial culture solution is set to be 0.1 m/s to 3.0 m/s and a filtration flux is set to be 0.1 $m^3/m^2/d$ to 2.0 $m^3/m^2/d$, and, when the backwashing is performed, a backwashing flux is set to be 1.0 $m^3/m^2/d$ to 10.0 $m^3/m^2/d$, and
after the step a-1 and the step a-2 are repeated at least once, the step proceeds to the step b.

4. The method for filtrating a microbial culture solution using a membrane module according to any one of claims 1 to 3, wherein the chemical solution has a temperature of 20°C to 50°C.

5. The method for filtrating a microbial culture solution using a membrane module according to any one of claims 1 to 4, wherein the separation membrane contained in the membrane module is a separation membrane comprising a fluorine-based resin.

6. The method for filtrating a microbial culture solution using a membrane module according to any one of claims 1 to 5, wherein the microbial culture solution is beer and the separation membrane contained in the membrane module has an average pore diameter of 0.3 $\mu$m to 1.0 $\mu$m.

7. A method for filtrating a microbial culture solution using a membrane module, the method comprising:

(a) a step of performing cross-flow filtration of the microbial culture solution using the membrane module;
(b) a step of performing water rinsing of the membrane module after the step a;
(c) a step of bringing the membrane module into contact with a chemical solution after the step b; and
(d) a step of performing water rinsing of the membrane module after the step c,
wherein a filtrate that has permeated through the membrane module by the cross-flow filtration in the step a has a total sugar concentration of 1,000 mg/L to 100,000 mg/L, determined according to the description and a protein concentration of 50 mg/L to 1,000 mg/L, determined according to the description,
the step c comprises:

(c-2) a step of bringing the membrane module into contact with a chemical solution containing hypochlorite; and
(c-3) a step of bringing the membrane module into contact with a chemical solution containing nonionic surfactant, and

the steps a to d are repeated in this order,
wherein, in the cross-flow filtration in the step a, a membrane surface linear velocity of the microbial culture solution is set to be 0.1 m/s to 3.0 m/s and a filtration flux is set to be 0.1 $m^3/m^2/d$ to 2.0 $m^3/m^2/d$,
wherein the hypochlorite has an effective chlorine concentration of 0.05% by mass to 1% by mass, and the nonionic surfactant has a nonionic surfactant concentration of 0.05% by mass to 3% by mass and a hydrophilic-lipophilic balance (HLB) of 13 to 17,
wherein the chemical solution has a pH of 10 to 14, and
wherein the step c has a step which is operated within a range where a product CT [(mg/L) · h] of a hypochlorite concentration C [mg/L] in the chemical solution and a contact time T [h] between the hypochlorite and a separation membrane contained in the membrane module satisfies Expression (1):

$$9.2 \times 10^5 F - 1400 \leq CT \leq 2.5 \, (1/\alpha) + 380 \qquad \text{Expression (1)}$$

in Expression (1), F represents a ratio of the protein concentration [mg/L] in the filtrate which has permeated through the membrane module by the cross-flow filtration in the step a with respect to a total organic carbon amount [mg/L] in the filtrate, and $\alpha$ represents an absolute value [1/h] of an attenuation rate of a membrane strength initial value ratio with respect to an immersion time in a case where the separation membrane is immersed in a Fenton's reagent which is a mixed solution of 5,000 ppm of $H_2O_2$ and 300 ppm of $Fe^{2+}$
the total organic carbon amount and the attenuation rate $\alpha$ being determined according to the description.


**Patentansprüche**

1. Verfahren zum Filtrieren einer mikrobiellen Kulturlösung unter Verwendung eines Membranmoduls, wobei das Verfahren umfasst:

   (a) einen Schritt des Durchführens einer Querstromfiltration der mikrobiellen Kulturlösung unter Verwendung des Membranmoduls;
   (b) einen Schritt des Durchführens einer Wasserspülung des Membranmoduls nach Schritt a;
   (c) einen Schritt des In-Kontakt-Bringens des Membranmoduls mit einer chemischen Lösung nach Schritt b; und
   (d) einen Schritt des Durchführens einer Wasserspülung des Membranmoduls nach Schritt c,
   wobei ein Filtrat, das durch die Querstromfiltration in Schritt a durch das Membranmodul hindurchgetreten ist, eine Gesamtzuckerkonzentration von 1.000 mg/L bis 100.000 mg/L, bestimmt wie in der Beschreibung dargelegt, und eine Proteinkonzentration von 50 mg/L bis 1.000 mg/L, bestimmt wie in der Beschreibung dargelegt, aufweist,
   wobei Schritt c einen Schritt (c-1) des In-Kontakt-Bringens des Membranmoduls mit einer chemischen Lösung, die Hypochlorit und ein nichtionisches Tensid enthält, beinhaltet, und
   die Schritte a bis d in dieser Reihenfolge wiederholt werden, und
   wobei bei der Querstromfiltration in Schritt a eine lineare Membranoberflächengeschwindigkeit der mikrobiellen Kulturlösung auf 0,1 m/s bis 3,0 m/s und ein Filtrationsfluss auf 0,1 $m^3/m^2/d$ bis 2,0 $m^3/m^2/d$ eingestellt wird,
   wobei das Hypochlorit eine effektive Chlorkonzentration von 0,05 Masse-% bis 1 Masse-% aufweist, und das nichtionische Tensid eine Konzentration an nichtionischem Tensid von 0,05 Masse-% bis 3 Masse-% und ein hydrophil-lipophiles Gleichgewicht (HLB) von 13 bis 17 aufweist,
   wobei die chemische Lösung einen pH-Wert von 10 bis 14 aufweist, und
   wobei Schritt c einen Schritt aufweist, der innerhalb eines Bereichs durchgeführt wird, in dem ein Produkt CT [(mg/L) • h] aus einer Hypochlorit-Konzentration C [mg/L] in der chemischen Lösung und einer Kontaktzeit T [h] zwischen dem Hypochlorit und einer in dem Membranmodul enthaltenen Trennmembran den Ausdruck (1) erfüllt:

$$9,2 \times 10^5 F - 1400 \leq CT \leq 2,5 \, (1/\alpha) + 380 \qquad \text{Ausdruck (1)}$$

   wobei in Ausdruck (1) F ein Verhältnis der Proteinkonzentration [mg/L] in dem Filtrat, das durch das Membranmodul durch die Querstromfiltration in Schritt a durchgedrungen ist, in Bezug auf eine Gesamtmenge an organischem Kohlenstoff [mg/L] in dem Filtrat darstellt, und $\alpha$ einen absoluten Wert [1/h] einer Abschwächungsrate eines Membranfestigkeits-Anfangswert-Verhältnisses in Bezug auf eine Eintauchzeit in einem Fall darstellt, in dem die Trennmembran in ein Fenton-Reagenz eingetaucht wird, das eine gemischte Lösung aus 5000 ppm

$H_2O_2$ und 300 ppm $Fe^{2+}$ ist,
wobei die Gesamtmenge an organischem Kohlenstoff und die Abschwächungsrate $\alpha$ wie in der Beschreibung dargelegt bestimmt werden.

2. Verfahren zum Filtrieren einer mikrobiellen Kulturlösung unter Verwendung eines Membranmoduls nach Anspruch 1,

wobei in einem Fall, in dem Ausdruck (2) in Schritt a erfüllt ist, der Schritt mit Schritt b fortgesetzt wird:

$$5 \leq R/R_0 \leq 16 \qquad \text{Ausdruck (2)}$$

wobei in Ausdruck (2) R einen Filtrationswiderstandswert $[m^{-1}]$ während der Durchführung der Querstromfiltration der mikrobiellen Kulturlösung in Schritt a darstellt und Ro einen Filtrationswiderstandswert $[m^{-1}]$ zu Beginn der Querstromfiltration darstellt.

3. Verfahren zum Filtrieren einer mikrobiellen Kulturlösung unter Verwendung eines Membranmoduls nach Anspruch 1 oder 2,

wobei Schritt a Folgendes umfasst:

(a-1) einen Schritt des Durchführens einer Querstromfiltration der mikrobiellen Kulturlösung unter Verwendung des Membranmoduls; und
(a-2) einen Schritt des Durchführens einer Rückspülung des Membranmoduls,

wobei, wenn die Querstromfiltration durchgeführt wird, eine lineare Geschwindigkeit der Membranoberfläche der mikrobiellen Kulturlösung auf 0,1 m/s bis 3,0 m/s und ein Filtrationsfluss auf 0,1 $m^3/m^2$/d bis 2,0 $m^3/m^2$/d eingestellt wird, und, wenn die Rückspülung durchgeführt wird, ein Rückspülfluss auf 1,0 $m^3/m^2$/d bis 10,0 $m^3/m^2$/d eingestellt wird, und
nachdem Schritt a-1 und Schritt (a-2) zumindest einmal wiederholt wurden, der Schritt mit Schritt b fortgesetzt wird.

4. Verfahren zum Filtrieren einer mikrobiellen Kulturlösung unter Verwendung eines Membranmoduls nach einem der Ansprüche 1 bis 3,
wobei die chemische Lösung eine Temperatur von 20 °C bis 50 °C aufweist.

5. Verfahren zum Filtrieren einer mikrobiellen Kulturlösung unter Verwendung eines Membranmoduls nach einem der Ansprüche 1 bis 4,
wobei die im Membranmodul enthaltene Trennmembran eine Trennmembran ist, die ein Harz auf Fluorbasis umfasst.

6. Verfahren zum Filtrieren einer mikrobiellen Kulturlösung unter Verwendung eines Membranmoduls nach einem der Ansprüche 1 bis 5,
wobei die mikrobielle Kulturlösung Bier ist und die im Membranmodul enthaltene Trennmembran einen mittleren Porendurchmesser von 0,3 $\mu$m bis 1,0 $\mu$m aufweist.

7. Verfahren zum Filtrieren einer mikrobiellen Kulturlösung unter Verwendung eines Membranmoduls, wobei das Verfahren umfasst:

(a) einen Schritt des Durchführens einer Querstromfiltration der mikrobiellen Kulturlösung unter Verwendung des Membranmoduls;
(b) einen Schritt des Durchführens einer Wasserspülung des Membranmoduls nach Schritt a;
(c) einen Schritt des In-Kontakt-Bringens des Membranmoduls mit einer chemischen Lösung nach Schritt b; und
(d) einen Schritt des Durchführens einer Wasserspülung des Membranmoduls nach Schritt c,

wobei ein Filtrat, das durch die Querstromfiltration in Schritt a durch das Membranmodul hindurchgetreten ist, eine Gesamtzuckerkonzentration von 1.000 mg/L bis 100.000 mg/L, bestimmt wie in der Beschreibung dargelegt, und eine Proteinkonzentration von 50 mg/L bis 1.000 mg/L aufweist, bestimmt wie in der Beschreibung dargelegt,

wobei Schritt c umfasst:

(c-2) einen Schritt des In-Kontakt-Bringens des Membranmoduls mit einer chemischen Lösung, die Hypochlorit beinhaltet; und
(c-3) einen Schritt des In-Kontakt-Bringens des Membranmoduls mit einer chemischen Lösung, die ein nichtionisches Tensid enthält; und

wobei die Schritte a bis d in dieser Reihenfolge wiederholt werden,
wobei bei der Querstromfiltration in Schritt a eine lineare Membranoberflächengeschwindigkeit der mikrobiellen Kulturlösung auf 0,1 m/s bis 3,0 m/s und ein Filtrationsfluss auf 0,1 $m^3/m^2/d$ bis 2,0 $m^3/m^2/d$ eingestellt wird,
wobei das Hypochlorit eine effektive Chlorkonzentration von 0,05 Masse-% bis 1 Masse-% aufweist, und das nichtionische Tensid eine Konzentration an nichtionischem Tensid von 0,05 Masse-% bis 3 Masse-% und ein hydrophil-lipophiles Gleichgewicht (HLB) von 13 bis 17 aufweist,
wobei die chemische Lösung einen pH-Wert von 10 bis 14 aufweist, und
wobei der Schritt c einen Schritt aufweist, der innerhalb eines Bereichs durchgeführt wird, in dem ein Produkt CT [(mg/L) • h] aus einer Hypochlorit-Konzentration C [mg/L] in der chemischen Lösung und einer Kontaktzeit T [h] zwischen dem Hypochlorit und einer in dem Membranmodul enthaltenen Trennmembran den Ausdruck (1) erfüllt:

$$9,2 \times 10^5 F - 1400 \leq CT \leq 2,5 \, (1/\alpha) + 380 \qquad \text{Ausdruck (1)}$$

wobei in Ausdruck (1) F ein Verhältnis der Proteinkonzentration [mg/L] in dem Filtrat, das durch das Membranmodul durch die Querstromfiltration in Schritt a durchgedrungen ist, in Bezug auf eine Gesamtmenge an organischem Kohlenstoff [mg/L] in dem Filtrat darstellt, und $\alpha$ einen absoluten Wert [1/h] einer Abschwächungsrate eines Membranfestigkeits-Anfangswert-Verhältnisses in Bezug auf eine Eintauchzeit in einem Fall darstellt, in dem die Trennmembran in ein Fenton-Reagenz eingetaucht wird, das eine gemischte Lösung aus 5000 ppm $H_2O_2$ und 300 ppm $Fe^{2+}$ ist,
wobei die Gesamtmenge an organischem Kohlenstoff und die Abschwächungsrate $\alpha$ wie in der Beschreibung dargelegt bestimmt werden.

## Revendications

1. Procédé de filtration d'une solution de culture microbienne utilisant un module membranaire, le procédé comprenant :

(a) une étape de réalisation d'une filtration tangentielle de la solution de culture microbienne en utilisant le module membranaire ;
(b) une étape de réalisation d'un rinçage à l'eau du module membranaire après l'étape a ;
(c) une étape de mise en contact du module membranaire avec une solution chimique après l'étape b ; et
(d) une étape de réalisation d'un rinçage à l'eau du module membranaire après l'étape c ;
dans lequel un filtrat qui a traversé le module membranaire par la filtration tangentielle dans l'étape a a une concentration totale en sucres de 1 000 mg/l à 100 000 mg/l, déterminée selon la description et une concentration en protéines de 50 mg/l à 1 000 mg/l, déterminée selon la description,
l'étape c inclut une étape (c-1) de mise en contact du module membranaire avec une solution chimique contenant de l'hypochlorite et un tensioactif non ionique, et
les étapes a à d sont répétées dans cet ordre, et
dans lequel, dans la filtration tangentielle dans l'étape a, une vitesse linéaire de surface membranaire de la solution de culture microbienne est fixée pour être de 0,1 m/s à 3,0 m/s et un flux de filtration est fixé pour être de 0,1 $m^3/m^2/j$ à 2,0 $m^3/m^2/j$,
dans lequel l'hypochlorite a une concentration en chlore effective de 0,05 % en masse à 1 % en masse, et le tensioactif non ionique a une concentration en tensioactif non ionique de 0,05 % en masse à 3 % en masse et un équilibre hydrophile-lipophile (HLB) de 13 à 17,
dans lequel la solution chimique a un pH de 10 à 14, et
dans lequel l'étape c a une étape qui est effectuée dans une plage où un produit CT [(mg/l) • h] d'une concentration en hypochlorite C [mg/l] dans la solution chimique et d'un temps de contact T [h] entre l'hypochlorite et une membrane de séparation contenue dans le module membranaire satisfait à l'Expression (1) :

$$9,2 \times 10^5 F - 1400 \leq CT \leq 2,5 \, (1 \, /\alpha) + 380 \quad \text{Expression (1)}$$

dans l'Expression (1), F représente un rapport de la concentration en protéines [mg/l] dans le filtrat qui a traversé le module membranaire par la filtration tangentielle dans l'étape a par rapport à une quantité totale de carbone organique [mg/l] dans le filtrat, et $\alpha$ représente une valeur absolue [1/h] d'un taux d'atténuation d'un rapport de valeur initiale de résistance de membrane par rapport à un temps d'immersion dans un cas où la membrane de séparation est immergée dans un réactif de Fenton qui est une solution mélangée de 5 000 ppm de $H_2O_2$ et 300 ppm de $Fe^{2+}$,

la quantité totale de carbone organique et le taux d'atténuation $\alpha$ étant déterminés selon la description.

2. Procédé de filtration d'une solution de culture microbienne utilisant un module membranaire selon la revendication 1,

   dans lequel dans un cas où l'Expression (2) est satisfaite dans l'étape a, l'étape passe à l'étape b :

$$5 \leq R/R_0 \leq 16 \quad \text{Expression (2)}$$

   dans l'Expression (2), R représente une valeur de résistance à la filtration [m$^{-1}$] pendant la réalisation de la filtration tangentielle de la solution de culture microbienne dans l'étape a, et $R_0$ représente une valeur de résistance à la filtration [m$^{-1}$] à un début de la filtration tangentielle.

3. Procédé de filtration d'une solution de culture microbienne utilisant un module membranaire selon la revendication 1 ou 2,

   dans lequel l'étape a comprend :

      (a-1) une étape de réalisation d'une filtration tangentielle de la solution de culture microbienne en utilisant le module membranaire ; et
      (a-2) une étape de réalisation d'un lavage à contre-courant du module membranaire,

   lorsque la filtration tangentielle est réalisée, une vitesse linéaire de surface membranaire de la solution de culture microbienne est fixée pour être de 0,1 m/s à 3,0 m/s et un flux de filtration est fixé pour être de 0,1 m$^3$/m$^2$/j à 2,0 m$^3$/m$^2$/j, et, lorsque le lavage à contre-courant est réalisé, un flux de lavage à contre-courant est fixé pour être de 1,0 m$^3$/m$^2$/j à 10,0 m$^3$/m$^2$/j, et
   après que l'étape a-1 et l'étape a-2 ont été répétées au moins une fois, l'étape passe à l'étape b.

4. Procédé de filtration d'une solution de culture microbienne utilisant un module membranaire selon l'une quelconque des revendications 1 à 3,
   dans lequel la solution chimique a une température de 20 °C à 50 °C.

5. Procédé de filtration d'une solution de culture microbienne utilisant un module membranaire selon l'une quelconque des revendications 1 à 4,
   dans lequel la membrane de séparation contenue dans le module membranaire est une membrane de séparation comprenant une résine à base de fluor.

6. Procédé de filtration d'une solution de culture microbienne utilisant un module membranaire selon l'une quelconque des revendications 1 à 5,
   dans lequel la solution de culture microbienne est de la bière et la membrane de séparation contenue dans le module membranaire a un diamètre moyen de pore de 0,3 $\mu$m à 1,0 $\mu$m.

7. Procédé de filtration d'une solution de culture microbienne utilisant un module membranaire, le procédé comprenant :

      (a) une étape de réalisation d'une filtration tangentielle de la solution de culture microbienne en utilisant le module membranaire ;
      (b) une étape de réalisation d'un rinçage à l'eau du module membranaire après l'étape a ;
      (c) une étape de mise en contact du module membranaire avec une solution chimique après l'étape b) ; et
      (d) une étape de réalisation d'un rinçage à l'eau du module membranaire après l'étape c ;

   dans lequel un filtrat qui a traversé le module membranaire par la filtration tangentielle dans l'étape a a une concentration totale en sucres de 1 000 mg/l à 100 000 mg/l, déterminée selon la description et une concentration en protéines de 50 mg/l à 1 000 mg/l, déterminée selon la description,

l'étape c comprend :

(c-2) une étape de mise en contact du module membranaire avec une solution chimique contenant de l'hypochlorite ; et
(c-3) une étape de mise en contact du module membranaire avec une solution chimique contenant un tensioactif non ionique, et

les étapes a à d sont répétées dans cet ordre,
dans lequel, dans la filtration tangentielle dans l'étape a, une vitesse linéaire de surface membranaire de la solution de culture microbienne est fixée pour être de 0,1 m/s à 3,0 m/s et un flux de filtration est fixé pour être de 0,1 m$^3$/m$^2$/j à 2,0 m$^3$/m$^2$/j,
dans lequel l'hypochlorite a une concentration en chlore effective de 0,05 % en masse à 1 % en masse, et le tensioactif non ionique a une concentration en tensioactif non ionique de 0,05 % en masse à 3 % en masse et un équilibre hydrophile-lipophile (HLB) de 13 à 17,
dans lequel la solution chimique a un pH de 10 à 14, et
dans lequel l'étape c a une étape qui est effectuée dans une plage où un produit CT [(mg/l)•h] d'une concentration en hypochlorite C [mg/l] dans la solution chimique et d'un temps de contact T [h] entre l'hypochlorite et une membrane de séparation contenue dans le module membranaire satisfait à l'Expression (1) :

$$9,2 \times 10^5 F - 1400 \leq CT \leq 2,5 (1/\alpha) + 380 \quad \text{Expression (1)}$$

dans l'Expression (1), F représente un rapport de la concentration en protéines [mg/l] dans le filtrat qui a traversé le module membranaire par la filtration tangentielle dans l'étape a par rapport à une quantité totale de carbone organique [mg/l] dans le filtrat, et $\alpha$ représente une valeur absolue [1/h] d'un taux d'atténuation d'un rapport de valeur initiale de résistance de membrane par rapport à un temps d'immersion dans un cas où la membrane de séparation est immergée dans un réactif de Fenton qui est une solution mélangée de 5 000 ppm de H$_2$O$_2$ et 300 ppm de Fe$^{2+}$,
la quantité totale de carbone organique et le taux d'atténuation $\alpha$ étant déterminés selon la description.

Fig. 1

Fig. 2

cross-flow filtration (step a)

No ← condition for proceeding to the next step $R/R_0$

Yes

water rinsing (step b)

chemical solution washing (step c)

water rinsing (step d)

Fig. 3

CT value [(mg/L)·h]

$CT=3.8 \times 10^6 F-5700$

$CT=9.2 \times 10^5 F-1400$

F
**(protein concentration** [mg/L]**/ total organic carbon amount** [mg/L]**)**

Fig. 4

membrane breaking strength $S_t$

$S_{A0}$
$S_{A1}$
$S_{B0}$

$S_{B1}$

Membrane A

Membrane B

$0$

$t_1$

immersion time in Fenton's reagent t [h]

## Fig. 5

attenuation rate $\alpha_A = |l_A/t_A|$

attenuation rate $\alpha_B = |l_B/t_B|$

membrane strength initial value ratio $S_t/S_0$

immersion time in Fenton's reagent t [h]

## Fig. 6

$CT = 2.5(1/\alpha) + 380$

$CT = 1.4(1/\alpha) + 240$

$CT = 0.56(1/\alpha) + 95$

CT value $[(mg/L) \cdot h]$

$1/\alpha$ [hr]

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2013031839 A
- JP 2010535097 T
- US 2013330792 A1
- WO 02092202 A1